(19) Europäisches Patentamt
European Patent Office
Office européen des brevets

(11) Numéro de publication: **0 233 793 B1**

(12) **FASCICULE DE BREVET EUROPEEN**

(45) Date de publication de fascicule du brevet: **01.04.92**

(51) Int. Cl.5: **C07D 215/40**, C07D 401/06, C07D 409/06, C07D 417/06, C07D 413/06, C07D 405/06, A61K 31/47, C07D 215/18

(21) Numéro de dépôt: **87400033.4**

(22) Date de dépôt: **09.01.87**

# (54) Dérivés de la décahydroquinoléine, leur procédé de préparation, les intermédiaires de préparation, leur application à titre de médicaments et les compositions les renfermant.

(30) Priorité: **13.01.86 FR 8600354**

(43) Date de publication de la demande:
**26.08.87 Bulletin 87/35**

(45) Mention de la délivrance du brevet:
**01.04.92 Bulletin 92/14**

(84) Etats contractants désignés:
**AT BE CH DE ES FR GB GR IT LI LU NL SE**

(56) Documents cités:
**DE-A- 2 656 678**
**FR-A- 2 370 722**

(73) Titulaire: **ROUSSEL-UCLAF**
**35, Boulevard des Invalides**
**F-75007 Paris(FR)**

(72) Inventeur: **Clemence, François**
**2, rue Turgot**
**F-75009 Paris(FR)**
Inventeur: **Fortin, Michel**
**12, Passage Cottin**
**F-75018 Paris(FR)**
Inventeur: **Le Martret, Odile**
**42, Avenue de Versailles**
**F-75016 Paris(FR)**
Inventeur: **Delevallee, Françoise**
**48-50, Avenue de la Dame Blache**
**F-94120 Fontenay-sous-Bois(FR)**

(74) Mandataire: **Bourgouin, André et al**
**Département des Brevets ROUSSEL UCLAF**
**B.P. no 9**
**F-93230 Romainville(FR)**

EP 0 233 793 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention concerne de nouveaux dérivés de la décahydroquinoléine, leur procédé de préparation, leur application à titre de médicaments, les compositions pharmaceutiques les renfermant et les nouveaux intermédiaires obtenus.

L'invention a pour objet des composés de formule (I) :

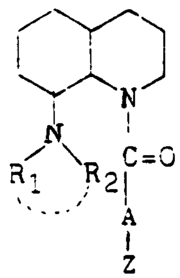

(I)

dans laquelle $R_1$ et $R_2$, identiques ou différents, représentent un atome d'hydrogène ou un radical alcoyle renfermant de 1 à 5 atomes de carbone ou $R_1$ et $R_2$ forment ensemble avec l'atome d'azote auquel ils sont liés un radical pyrrolidinyle, pipérazinyle, pipéridinyle ou morpholinyle, ces radicaux étant éventuellement substitués par un radical alcoyle ou alcoxy renfermant de 1 à 5 atomes de carbone, A représente une chaîne $(CH_2)_n$ dans laquelle n représente un nombre de 0 à 5, ou A représente une chaîne alcoylène substituée par un radical alcoyle renfermant au total de 2 à 8 atomes de carbone, Z représente un radical phényle éventuellement substitué par un ou plusieurs radicaux identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z représente un radical naphtyle, un radical indényle, pyridinyle, thiényle, thiazolyle, oxazolyle, isoxazolyle, imidazolyle, indolyle, quinolyle, benzofuranyle, benzo [b] thiényle, benzimidazolyle, benzoxazolyle ou benzothiazolyle tous ces radicaux étant éventuellement substitués par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes, lesdits composés de formule (I) pouvant être dans toutes les formes énantioméres et diastéréoisomères possibles et sous forme de sels d'addition avec les acides ou de sels d'ammonium quaternaire.

Lorsque $R_1$ et $R_2$ représentent un radical alcoyle, il s'agit de préférence d'un radical méthyle, éthyle, n-propyle ou isopropyle.

Par substituant alcoyle, alcoxy ou halogène, on entend de préférence méthyle, éthyle, propyle ou butyle linéaire ou ramifié, méthoxy, éthoxy, propoxy ou butoxy linéaire ou ramifié, fluoro, chloro, bromo ou iodo.

Dans les valeurs monoalkyl et dialkylamino, les radicaux alkyles sont préférentiellement les radicaux méthyle ou éthyle.

Lorsque A représente une chaîne $(CH_2)_n$, n est de préférence égal à 0 ou 1.

Lorsque A représente une chaîne alcoylène substituée par un radical alcoyle, par alcoyle on entend de préférence méthyle ou éthyle et A est alors de préférence un radical 1,1-éthanediyl, 1-méthyl 1,2-éthanediyl, 1-méthyl ou 2-méthyl 1,3-propanediyl, 1-éthyl 1,2-éthanediyl.

Par ailleurs, un composé de formule (I) peut exister sous la forme de quatre racémates, ou paire d'énantiomères. Les énantiomères de chaque paire peuvent être séparés par des procédés classiques. L'invention couvre donc toutes les formes énantiomères et diastéréoisomères des composés de formule (I).

Les sels d'addition avec les acides minéraux ou organiques peuvent être, par exemple, les sels formés avec les acides chlorhydrique, bromhydrique, nitrique, sulfurique, phosphorique, acétique, propionique, formique, benzoïque, maléique, fumarique, succinique, tartrique, citrique, oxalique, glyoxylique, aspartique, alcanesulfoniques tels que l'acide méthane sulfonique et arylsulfoniques, tels que l'acide benzène sulfonique.

L'invention concerne aussi des composés de formule (I) sous forme de sels d'ammonium quaternaire.

Par sels d'ammonium quaternaire, on entend les composés de formule (I) quaternisés par des produits

de type R-Y, R étant un radical alcoyle ayant de 1 à 4 atomes de carbone tel qu'un radical méthyle, éthyle, n-propyle ou isopropyle et Y un anion halogénure, par exemple, un chlorure, un bromure, un iodure.

L'invention a notamment pour objet les composés de formule (I) dans laquelle $R_1$ et $R_2$ représentent un radical méthyle ou éthyle, ou forment ensemble avec l'atome d'azote auquel ils sont liés, un radical pyrrolidinyle ou pipéridinyle, A représente une chaîne $(CH_2)_n$ où n est égal à 0 ou 1, ou une chaine 1,1-éthanediyl, Z représente un radical phényle, éventuellement substitué par un ou plusieurs radicaux identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z représente un radical naphtyle, indényle, pyridinyle, thiényle, thiazolyle, oxazolyle, isoxazolyle, imidazolyle, indolyle, quinolyle, benzofuranyle, benzo [b] thiényle, benzimidazolyle, benzoxazolyle ou benzothiazolyle éventuellement substitué par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes ainsi que leurs sels d'addition avec les acides et leurs sels d'ammonium quaternaire.

L'invention a plus particulièrement pour objet les composés de formule (I) dans laquelle $R_1$, $R_2$ et A sont définis comme ci-dessus, et Z représente un radical phényle, éventuellement substitué par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z représente un radical naphtyle, pyridinyle, thiényle, indolyle, benzo [b] thiényle éventuellement substitué par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes ainsi que leurs sels d'addition avec les acides et leurs sels d'ammonium quaternaire.

L'invention a tout particulièrement pour objet les composés de formule (I) dans laquelle $R_1$ et $R_2$ forment ensemble avec l'atome d'azote auquel ils sont liés, un radical pyrrolidinyle, A représente une chaîne $(CH_2)_n$ où n est égal à 0 ou 1, ou une chaîne 1,1-éthanediyl, Z représente un radical phényle substitué par un ou plusieurs substituants choisis dans le groupe constitué par les atomes d'halogènes et le radical trifluorométhyle ou Z représente un radical naphtyle ou benzo [b] thiényle, ainsi que leurs sels d'addition avec les acides et leurs sels d'ammonium quaternaire.

L'invention a tout particulièrement pour objet les composés de formule (I) dont les noms suivent :

- la (4a alpha, 8 alpha, 8a alpha) (±) décahydro 1-[(3,4-dichlorophényl) acétyl] 8-(1-pyrrolidinyl) quinoléine,
- la (4a alpha, 8 alpha, 8a alpha) (±) décahydro 1-[(4-(trifluorométhyl) phényl] acétyl] 8-(1-pyrrolidinyl) quinoléine,
- la (4a alpha, 8 alpha, 8a alpha) (±) décahydro 1-[(4-bromophényl) acétyl] 8-(1-pyrrolidinyl) quinoléine,
- la (4a alpha, 8 alpha, 8a alpha) (±) décahydro 1-[2-(3,4-dichloro-phényl) 1-oxopropyl] 8-(1-pyrrolidinyl) quinoléine,
- la (4a alpha, 8 alpha, 8a alpha) (±) décahydro 1-(3,4-dichlorobenzoyl) 8-(1-pyrrolidinyl) quinoléine,
- la (4a alpha, 8 alpha, 8a alpha) (±) décahydro 1-[(4-benzo [b] thiényl) acétyl] 8-(1-pyrrolidinyl) quinoléine,
- la (4a alpha, 8 alpha, 8a alpha) (±) décahydro 1-[(1-naphtalényl) acétyl] 8-(1-pyrrolidinyl) quinoléine,

ainsi que leurs sels d'addition avec les acides et leurs sels d'ammonium quaternaire.

L'invention concerne aussi un procédé caractérisé en ce que l'on condense la 8-chloro 5,6,7,8-tétrahydro quinoléine, de formule :

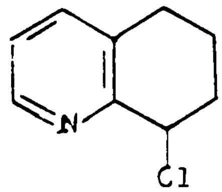

avec une amine de formule :

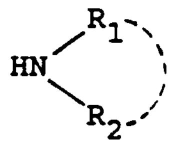

dans laquelle $R_1$ et $R_2$ ont les significations indiquées précédemment pour obtenir un composé de formule II :

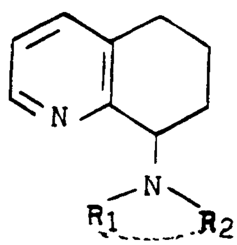

(II)

que l'on réduit pour obtenir un composé de formule III :

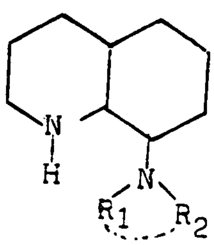

(III)

que l'on condense avec un composé de formule IV ou un dérivé fonctionnel de ce composé :

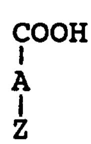

(IV)

dans laquelle A et Z ont les significations indiquées précédemment pour obtenir un composé de formule (I) sous toutes les formes énantiomères et diastéréoisomères possibles, que l'on traite, si désiré, avec un acide minéral ou organique pour obtenir un sel ou avec un halogénure d'alcoyle pour obtenir un sel d'ammonium quaternaire.

L'invention a plus particulièrement pour objet un procédé tel que défini précédemment caractérisé en

ce que si la réduction du composé de formule II est chimique, on obtient préférentiellement des composés de formule (I) dans laquelle la jonction du cycle est trans et si la réduction du composé de formule (II) est catalytique, on obtient préférentiellement des composés de formule (I) dans laquelle la jonction du cycle est cis.

Dans un mode de réalisation préférée du procédé de l'invention :

- l'agent de réduction chimique utilisé pour réduire le composé de formule (II) et permettant d'obtenir préférentiellement des composés de formule (I) dans laquelle la jonction du cycle est trans est le sodium dans l'éthanol.

On peut aussi utiliser un alcoolate dans un autre alcool.

- La réduction catalytique des composés de formule (II) pour obtenir préférentiellement les composés de formule (I) dans laquelle la jonction du cycle est cis, est une hydrogénation catalytique. Le catalyseur que l'on utilise est de préférence l'oxyde de platine.
- L'activation de la fonction carboxyle du composé de formule IV pour réaliser la condensation avec le composé de formule III, s'effectue en présence de carbonyl diimidazole ou de dicyclohexylcarbodiimide. On peut également activer l'acide de formule IV sous la forme d'un chlorure d'acide ou d'anhydride mixte.

Par ailleurs, les deux isomères correspondant aux orientations alpha ou béta du groupement :

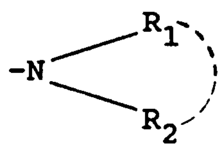

par rapport au cycle sont séparés par chromatographie ou par cristallisation fractionnée des sels pour les produits de formules II et III.

Chacun des racémiques obtenu peut être dédoublé par des méthodes usuelles, par exemple par séparation des sels des diastéréoisomères obtenus à partir d'acides optiquement actifs.

Les composés de formule I tels que définis ci-dessus ainsi que leurs sels d'addition avec les acides présentent d'intéressantes propriétés pharmacologiques. Ils présentent en particulier une forte affinité pour les récepteurs opiacés et notamment pour les récepteurs K et sont doués de propriétés analgésiques centrales.

Ils sont doués également des propriétés diurétiques.

En outre, certains d'entre eux possèdent des propriétés anti-arythmiques, anti ischémiques et hypotensives.

Ces propriétés justifient leur application en thérapeutique et l'invention a également pour objet à titre de médicaments, les produits tels que définis par la formule (I) ci-dessus ainsi que leurs sels d'addition avec les acides pharmaceutiquement acceptables et leurs sels d'ammonium quaternaire.

La présente invention a tout particulièrement pour objet, à titre de médicament, les produits préférés mentionnés précédemment et notamment :

- la (4a alpha, 8 alpha, 8a alpha) (±) décahydro 1-[(3,4-dichlorophényl) acétyl] 8-(1-pyrrolidinyl) quinoléine,
- la (4a alpha, 8 alpha, 8a alpha) (±) décahydro 1-[[4-(trifluorométhyl) phényl] acétyl] 8-(1-pyrrolidinyl) quinoléine,
- la (4a alpha, 8 alpha, 8a alpha) (±) décahydro 1-[(4-bromophényl) acétyl] 8-(1-pyrrolidinyl) quinoléine,
- la (4a alpha, 8 alpha, 8a alpha) (±) décahydro 1-[2-(3,4-dichloro-phényl) 1-oxopropyl] 8-(1-pyrrolidinyl) quinoléine,
- la (4a alpha, 8 alpha, 8a alpha) (±) décahydro 1-(3,4-dichlorobenzoyl) 8-(1-pyrrolidinyl) quinoléine,
- la (4a alpha, 8 alpha, 8a alpha) (±) décahydro 1-[(4-benzo [b] thiényl) acétyl) 8-(1-pyrrolidinyl) quinoléine,
- la (4a alpha, 8 alpha, 8a alpha) (±) décahydro 1-[(1-naphtalényl) acétyl] 8-(1-pyrrolidinyl) quinoléine,

ainsi que leurs sels d'addition avec les acides pharmaceutiquement acceptables et leurs sels d'ammonium quaternaire.

Les médicaments, objet de l'invention, permettent notamment de soulager une douleur quelle qu'en soit l'origine, par exemple une douleur de nature musculaire, articulaire ou nerveuse.

Ils peuvent être aussi utilisés dans le traitement des douleurs dentaires, des migraines, du zona dans le traitement des douleurs intenses, en particulier rebelles aux antalgiques périphériques, par exemple au cours des processus néoplasiques, dans le traitement des pancréatites, coliques néphrétiques ou biliaires,

dans le traitement des douleurs post-opératoires et post-traumatiques.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 20 et 400 mg de principe actif par jour, par voie orale et entre 5 et 100 mg par jour, par voie parentérale.

La 8-chloro 5,6,7,8-tétrahydro quinoléine, utilisée comme produit de départ dans le procédé de l'invention est préparée par chloruration de la 5,6,7,8-tétrahydroquinoléine N-oxyde selon la méthode indiquée dans le brevet US 3.991.065.

Ce composé n'est cependant pas décrit. L'invention a donc aussi pour objet la 8-chloro 5,6,7,8-tétrahydroquinoléine, à titre de produit industriel nouveau.

Les composés de formules II et III sont des produits chimiques nouveaux ; l'invention a donc pour objet ces produits à titre de produits industriels nouveaux.

Les exemples suivants illustrent l'invention sans toutefois la limiter.

**Exemple 1 : chlorhydrate de la (4a alpha, 8 alpha, 8a alpha) (±) décahydro 1-[(3,4-dichlorophényl)-acétyl] 8-(1-pyrrolidinyl) quinoléine.**

**Stade A : 8-pyrrolidinyl 5,6,7,8-tétrahydro quinoléine.**

On ajoute sous agitation en 7 minutes, en laissant chauffer 50 ml de pyrrolidine dans une solution renfermant 20 g de chlorhydrate de 8-chloro 5,6,7,8-tétrahydroquinoléine (préparation donnée à la fin de l'exemple 1) dans 50 ml d'eau.

La température atteint 57° C en fin d'introduction, on agite 1 heure 30 minutes à cette température. On laisse la température revenir à 20° C, sature par 17 g de chlorure de sodium, extrait à l'éther, sèche les phases organiques réunies, élimine les solvants sous pression réduite. On obtient 20,22 g de produit attendu sous la forme d'une huile.

**Stade B :** (4a alpha, 8 alpha, 8a alpha) (±) décahydro 8(1-pyrrolidinyl) quinoléine.

Ce produit est obtenu en mélange avec d'autres diastéréoisomères par réduction du produit obtenu au stade A précédent soit par hydrogénation catalytique soit par un mélange sodium - éthanol.

La description de ces deux réductions est donnée à la fin de la partie expérimentale.

**Stade C :** chlorhydrate de la (4a alpha, 8 alpha, 8a alpha) (±) décahydro 1-[(3,4-dichlorophényl) acétyl] 8-(1-pyrrolidinyl) quinoléine.

On agite durant une heure à 20-25° C, une solution renfermant 873 mg d'acide 3,4-dichlorophényl acétique, 690 mg de carbonyldiimidazole dans du tétrahydrofuranne, on ajoute ensuite 645 mg de produit obtenu au stade B en solution dans 3 ml de tétrahydrofuranne.

On agite 4 heures à température ambiante, élimine le tétrahydrofuranne sous pression réduite à moins de 40° C, reprend le résidu par 20 ml d'éther, lave la solution par une solution saturée de bicarbonate de sodium puis par de l'eau saturée en chlorure de sodium, sèche la phase éthérée et distille à sec sous pression réduite.

On obtient 1,39 g de produit brut, sous forme de base.

Préparation du chlorhydrate.

On dissout 1,298 g de ce dernier dans 5 ml d'éther, filtre, rince à l'éther, ajoute 2 ml d'éthanol au filtrat et ajoute 1,25 ml d'une solution éthanolique d'acide chlorhydrique (5,75N) jusqu'à obtention d'un pH = 1,2.

On amorce la cristallisation, laisse 2 heures à 20-22°C, essore, rince par un mélange d'éthanol-éther (3-1) et par de l'éther. on sèche sous pression réduite à 60°C et obtient 852 mg de chlorhydrate. On recristallise 825 mg de ce dernier dans l'éthanol et obtient 722 mg de produit attendu fondant à 233°C.

| Analyse : | | | | |
|---|---|---|---|---|
| Calculé :<br>Trouvé : | C% 58,41<br>58,7 | H% 6,77<br>7,0 | N% 6,48<br>6,5 | Cl% 24,63<br>24,6. |

Préparation de la 8-chloro 5,6,7,8-tétrahydro quinoléine (chlorhydrate).

On ajoute lentement à température ambiante 3 ml de chlorure de méthane sulfonyle à 1,49 g de 5,6,7,8-tétrahydro quinoléine N-oxyde sous agitation et atmosphère inerte.

On chauffe ensuite 4 heures à 80-82°C puis refroidit à 20°C. On verse sur 20 ml de solution saturée de bicarbonate de sodium puis ajoute lentement jusqu'à obtention d'un pH alcalin du bicarbonate de sodium. On extrait par du chlorure de méthylène, lave à l'eau, sèche les solutions organiques réunies, distille à sec sous pression réduite et obtient 1,53 g de produit attendu sous forme d'une huile.

Préparation du chlorhydrate.

On dissout l'huile ci-dessus dans 2 ml d'éthanol, ajoute 2 ml de solution éthanolique d'acide chlorhydrique (5,75N). Le chlorhydrate cristallise. On dilue lentement à 20-25°C par 4 ml d'éther, essore, rince par un mélange éthanol-éther (1-1) puis par de l'éther, sèche sous pression réduite à 20°C. Après recristallisation dans l'éthanol, on obtient 0,893 g de produit attendu. F = 240°C.

**Exemple 2 : oxalate de la (4a alpha, 8 béta, 8a alpha) (±) décahydro 1-[(3,4-dichlorophényl) acétyl] 8-(1-pyrrolidinyl) quinoléine.**

En opérant comme indiqué au stade C de l'exemple 1 à partir de 876 mg de (4a alpha, 8 béta, 8a alpha) (±) décahydro 8-(1-pyrrolidinyl) quinoléine (préparation donnée à la fin de la partie expérimentale), on obtient 1,660 g de produit.

Après chromatographie sur silice (éluant : acétate d'éthyle à 1 % de triéthylamine, on obtient 316 mg de produit attendu fondant à 90°C sous forme de base.

Préparation de l'oxalate.

On dissout 280 mg du produit ci-dessus dans 1,5 ml d'éthanol 100, filtre, rince à l'éthanol et ajoute au filtrat 130 mg d'acide oxalique.

On dilue lentement la solution obtenue par 6 ml d'éther, amorce la cristallisation, laisse une heure à température ambiante, essore, sèche sous pression réduite à 65°C et obtient 319 mg de produit. On recristallise 286 mg dans l'éthanol et obtient 223 mg de produit attendu fondant à 140°C.

| Analyse : | | | | |
|---|---|---|---|---|
| Calculé :<br>Trouvé : | C% 54,34<br>54,5 | H% 5,89<br>5,9 | N% 5,28<br>5,4 | Cl% 13,37<br>13,2. |

**Exemple 3 : chlorhydrate de la (4a alpha, 8 alpha, 8a béta) (±) décahydro 1-[(3,4-dichlorophényl) acétyl] 8-(1-pyrrolidinyl) quinoléine.**

On agite durant 1 heure à 20-22°C une solution renfermant 1,98 g d'acide 3,4-dichlorophényl acétique, 1,56 g de carbonyl diimidazole, dans 17 ml de tétrahydrofuranne. On ajoute ensuite 1,679 g de (4a alpha, 8 alpha, 8a béta) (±) décahydro 8-(1-pyrrolidinyl) quinoléine (préparation donnée à la fin de la partie

expérimentale) en solution dans 5 ml de tétrahydrofuranne.

On agite la solution à 20-22° C durant 4 heures, élimine le tétrahydrofuranne sous pression réduite à moins de 45° C. On empâte le résidu obtenu dans 15 ml d'éther et dans 5 ml d'une solution saturée de bicarbonate de sodium, essore, rince par de l'eau puis par de l'éther, sèche sous pression réduite et obtient 2,320 g de produit attendu sous forme de base fondant à 138° C.

Préparation du chlorhydrate

On dissout au reflux 2,309 g de base brute dans 2 ml d'éther, ajoute 2 ml d'une solution éthanolique d'acide chlorhydrique (5,75N), filtre à chaud, le produit cristallise au refroidissement dans le filtrat, on essore les cristaux, rince par de l'éthanol et de l'éther, sèche sous pression réduite à 65-70° C et obtient 1,816 g de produit attendu fondant à 214° C.

| Analyse : | C % | H % | N % | Cl % |
|---|---|---|---|---|
| Calculé : | 58,41 | 6,77 | 6,48 | 24,63 |
| Trouvé : | 58,6 | 6,8 | 6,6 | 24,6. |

**Exemple 4 : Fumarate de la (4a alpha, 8 béta, 8a béta) (±) décahydro 1-[(3,4-dichlorophényl) acétyl] 8-(1-pyrrolidinyl) quinoléine.**

On obtient 1,384 g de produit en opérant comme indiqué au stade C de l'exemple 1 à partir de 707 mg de (4a alpha, 8 béta, 8a béta) (± décahydro 8-(1-pyrrolidinyl) quinoléine (préparation donnée à la fin de la partie expérimentale).

L'huile obtenue est ensuite empâtée dans 10 ml de n-hexane. On amorce la cristallisation, essore, rince par du n-hexane, sèche sous pression réduite à 20° C et obtient 746 mg de produit attendu sous forme de base. F = 82-84° C.

Préparation du fumarate.

A 60° C, on dissout 817 mg de produit obtenu comme ci-dessus dans 8 ml d'éthanol, filtre, rince par de l'éthanol bouillant, ajoute 285 mg d'acide fumarique au filtrat, porte au reflux sous agitation. On laisse cristalliser par refroidissement, essore, rince par de l'éthanol et par de l'éther, sèche sous pression réduite à 70° C et obtient 0,49 mg de produit fondant à 220° C.

| Analyse : | C % | H % | N % | Cl % |
|---|---|---|---|---|
| Calculé : | 58,71 | 6,31 | 5,48 | 13,86 |
| Trouvé : | 58,4 | 6,3 | 5,4 | 13,7. |

Préparation des 4 diastéréoisomères suivants à utiliser pour la préparation des exemples 1 à 4.

- (4a alpha, 8 alpha, 8a alpha) (±) décahydro 8-(1-pyrrolidinyl) quinoléine = diastéréoisomère (cis A),
- (4a alpha, 8 béta, 8a alpha) (±) décahydro 8-(1-pyrrolidinyl) quinoléine = diastéréoisomère (cis B),
- (4a alpha, 8 alpha, 8a béta) (±) décahydro 8-(1-pyrrolidinyl) quinoléine = diastéréoisomère (trans A),
- (4a alpha, 8 béta, 8a béta) (±) décahydro 8-(1-pyrrolidinyl) quinoléine = diastéréoisomère (trans B).

Ces 4 diastéréoisomères sont obtenus par réduction de la 8-pyrrolidinyl 5,6,7,8-tétrahydro quinoléine préparée au stade A de l'exemple 1.

1) Réduction catalytique.

On introduit dans un appareil à hydrogéner 6,24 g de 8-pyrrolidinyl 5,6,7,8-tétrahydro quinoléine,
62 ml de méthanol,
6,2 ml d'acide chlorhydrique,
690 mg d'oxyde de platine.

On hydrogène à 22-25°C à la pression de 1850 mbars environ durant 17 heures ; l'absorption d'hydrogène dure environ 4 heures 30 minutes.

On filtre le catalyseur, rince et distille à sec sous pression réduite et obtient 8,67 g de produit de réduction.

a) Cristallisation du chlorhydrate de la (4a alpha, 8 alpha, 8a alpha) (±) décahydro 8-(1-pyrrolidinyl) quinoléine = diastéréoisomère (cis A).

On dissout la résine précédemment obtenue à 50-60°C dans 16 ml d'isopropanol et amorce la cristallisation à 20°C. On dilue ensuite par 12,5 ml d'éther, essore, rince par un mélange isopropanol-éther éthylique (1-1) puis par de l'éther, sèche sous pression réduite à 50°C, recristallise le produit obtenu dans 42,5 ml d'isopropanol à 2 % d'eau, essore, rince à l'isopropanol puis à l'éther et obtient 2,156 g de produit attendu sous forme de chlorhydrate fondant à 210°C.

b) Retour à la base.

On dissout 1 g de chlorhydrate dans 10 ml d'eau, ajoute 2 ml d'hydroxyde de sodium 2N, extrait à l'éther, décante, lave à l'eau saturée en chlorure de sodium. On sèche la solution éthérée et distille à sec sous pression réduite et obtient 0,680 g de produit attendu sous forme de base.

c) Chromatographie préparative des liqueurs-mères de cristallisation du chlorhydrate de la (4a alpha, 8 alpha, 8a alpha) (±) décahydro 8-(1-pyrrolidinyl) quinoléine = diastéréoisomère (cis A).

On groupe les liqueurs-mères de cristallisation du chlorhydrate de l'isomère cis A et effectue le retour à la base dans l'eau et l'acétate d'éthyle par de la lessive de soude. On extrait, lave à l'eau saturée en chlorure de sodium, sèche et distille à sec sous pression réduite. On effectue une chromatographie sur silice à pression ambiante (éluant : acétate d'éthyle 85 - méthanol 10 - triéthylamine 5). On recueille successivement les diastéréoisomères cis B, trans A, cis A.

- (4a alpha, 8 béta, 8a alpha) (±) décahydro 8-(1-pyrrolidinyl) quinoléine = diastéréoisomère (cis B)

On amène à sec sous pression réduite les fractions homogènes d'élution et obtient 2,059 g de produit attendu.

- (4a alpha, 8 alpha, 8a béta) (±) décahydro 8-(1-pyrrolidinyl) quinoléine = diastéréoisomère (trans A)

On amène à sec sous pression réduite les fractions suivantes et obtient 0,564 g de produit. On fait le chlorhydrate et le cristallise dans un mélange éthanol - éther (1-1) puis on effectue le retour à la base sur 201 mg de chlorhydrate dans l'eau, l'éther et l'hydroxyde de sodium 2N. On obtient 139 mg de produit attendu.

- (4a alpha, 8 alpha, 8a alpha) (±) décahydro 8-(1-pyrrolidinyl) quinoléine = diastéréoisomère (cis A)

On amène à sec sous pression réduite les éluants correspondants et obtient 0,686 g d'une résine brune qui donne un chlorhydrate fondant à 210°C.

2) Réduction chimique.

On porte au reflux à 20°C sous une pression de mercure de 2,5 à 3 cm, une solution renfermant 10,14 g de 8-pyrrolidinyl 5,6,7,8-tétrahydro quinoléine dans 400 ml d'éthanol puis introduit au reflux par petites fractions en 7 heures environ 18 g de sodium. On ramène à 20°C sous azote et laisse une nuit au repos, porte au reflux à nouveau et introduit par petits morceaux durant 5 heures, 14 g de sodium.

On ramène à 20°C sous azote et verse le mélange réactionnel sur 400 ml d'eau glacée sous agitation, élimine l'éthanol par distillation sous pression réduite à moins de 50°C, sature le milieu résiduel par 28 g de chlorure de sodium, extrait à l'éther, lave les phases éthérées par de l'eau saturée en chlorure de sodium, sèche, distille à sec sous pression réduite et obtient 6,68 g de produit brut attendu sous forme de mélange.

On effectue ensuite une :

chromatographie préparative pour séparer les diastéréoisomères suivants (4a alpha, 8 béta, 8a béta) (±) décahydro 8-(1-pyrrolidinyl) quinoléine = diastéréosiomère (trans B), (4a alpha, 8 alpha, 8a béta) (±) décahydro 8-(1-pyrrolidinyl) quinoléine = diastéréoisomère (trans A), (4a alpha, 8 alpha, 8a alpha) (±) décahydro 8-(1-pyrrolidinyl) quinoléine = diastéréoisomère (cis A),

On effectue une chromatographie sur silice (éluant : acétate d'éthyle 85 - méthanol 10 - triéthylamine 5).

- (4a alpha, 8 béta, 8a béta) (±) décahydro 8-(1-pyrrolidinyl) quinoléine = diastéréosiomère (trans B)

On amène à sec sous pression réduite les fractions renfermant le premier isomère et obtient 1,183 g d'une huile. On dissout 1,163 g de cette huile dans 2 ml d'éthanol, filtre, rince à l'éthanol puis ajoute 830 mg d'acide oxalique au filtrat. On dilue lentement la solution obtenue par 40 ml d'éther : un précipité se forme.

On décante la solution surnageante, lave la gomme à l'éther, la dissout dans 7 ml d'eau, ajoute 20 ml d'éther et 2 ml de lessive de soude.

On agite en ampoule, décante, lave par de l'eau saturée en chlorure de sodium, sèche les solutions éthérées, rince et distille à sec sous pression réduite.

On obtient 1,010 g de produit attendu.

- (4a alpha, 8 alpha, 8a béta) (±) décahydro 8-(1-pyrrolidinyl) quinoléine = diastéréoisomère (trans A)

On amène à sec sous pression réduite les fractions de chromatographie homogènes correspondant à l'isomère trans A et obtient 2,332 g d'une huile. On dissout 2,322 g de cette huile dans 2,5 ml d'éthanol, dilue le filtrat par 9,5 ml d'éther et ajoute 5 ml d'une solution d'acide chlorhydrique dans l'éthanol (5,75N). On essore le chlorhydrate obtenu, lave par un mélange d'éthanol et d'éther (1-1) et par de l'éther, sèche sous pression réduite à 60 °C et obtient 2,569 g de diastéréoisomère sous forme de chlorhydrate.

Le retour à la base s'effectue en traitant 2,464 g de chlorhydrate par 10 ml d'eau et 2 ml de lessive de soude à 32 %.

On agite, décante, réextrait à l'éther.

On lave les phases éthérées avec de l'eau saturée en chlorure de sodium, sèche distille à sec sous pression réduite et obtient 1,679 de produit attendu.

- (4a alpha, 8 alpha, 8a alpha) (±) décahydro 8-(1-pyrrolidinyl) quinoléine = diastéréoisomère (cis A)

On amène à sec sous pression réduite les fractions homogènes de chromatographie correspondant au diastéréoisomère cis A et obtient 0,579 g de produit attendu.

On dissout celui-ci dans 5 ml d'isopropanol, ajoute 2 ml de solution d'acide chlorhydrique sec dans l'isopropanol (4,4M) filtre, dilue par 7 ml d'éther, ajoute 0,15 ml d'eau, amorce la cristallisation, essore, rince par un mélange isopropanol - éther (1-1) et par de l'éther, sèche sous pression réduite à 60 °C et obtient 504 mg de diastéréoisomère cis A sous forme de chlorhydrate. F ≃ 210 °C.

On effectue le retour à la base sur 64 mg de chlorhydrate comme indiqué pour le chlorhydrate du diastéréoisomère trans A. On obtient 42 mg de produit attendu.

**Exemple 5 : Chlorhydrate de (4a alpha, 8 alpha, 8a alpha) (±) 1-[(4-chlorophényl)acétyl] décahydro 8-(1-pyrrolidinyl) quinoléine.**

On opère comme au stade C de l'exemple 1 à partir de 554 mg d'acide 4-chlorophényl acétique, 527 mg de carbonyldiimidazole et 520 mg de produit obtenu au stade A de l'exemple 1 en maintenant le milieu réactionnel sous agitation pendant 5 heures. Après cristallisation du chlorhydrate dans un mélange isopropanol-éther (1-1), on obtient 752 mg de produit attendu. F ≃ 222 °C (décomposition).

| Analyse : $C_{21}H_{29}ClN_2O$, HCl : 397,391 | | | | |
|---|---|---|---|---|
| Calculé : | C % 63,47 | H % 7,61 | N % 7,05 | Cl % 17,84 |
| Trouvé : | 63,6 | 7,6 | 6,8 | 17,8 |

**Exemple 6 : Chlorhydrate de (4a alpha, 8 alpha, 8a alpha) (±) 1-[(4-(trifluorométhyl) phényl) acétyl]**

**décahydro 8-(1-pyrrolidinyl) quinoléine.**

On opère comme à l'exemple précédent en utilisant 663 mg d'acide 4-trifluorométhylphényl acétique et en maintenant l'agitation pendant 16 heures. Après cristallisation du chlorhydrate dans l'éthanol, on obtient 921 mg de produit attendu. F ≃ 208 ° C (décomposion).

| Analyse : $C_{22}H_{29}F_3N_2O$, HCl : 430,944 | | | | | |
|---|---|---|---|---|---|
| Calculé :<br>Trouvé : | C% 61,32<br>61,4 | H% 7,02<br>7,1 | N% 6,50<br>6,4 | Cl% 8,23<br>8,1 | F% 13,22<br>12,9 |

**Exemple 7 : Chlorhydrate de (4a alpha, 8 alpha, 8a alpha) (±) 1-[(4-bromophényl) acétyl] décahydro 8-(1-pyrrolidinyl) quinoléine.**

On opère comme à l'exemple 5 en utilisant 699 mg d'acide 4-bromophényl acétique en maintenant l'agitation pendant 20 heures. Après cristallisation du chlorhydrate dans l'isopropanol, on obtient 685 mg de produit attendu. F ≃ 235 ° C (décomposition).

| Analyse : $C_{21}H_{29}BrN_2O$, HCl : 441,847 | | | | | |
|---|---|---|---|---|---|
| Calculé :<br>Trouvé : | C% 57,09<br>57,4 | H% 6,84<br>6,9 | N% 6,34<br>6,3 | Cl% 8,02<br>7,8 | Br% 18,08<br>18,0 |

**Exemple 8 : Chlorhydrate de (4a alpha, 8 alpha, 8a alpha) (±) 1-[(4-nitrophéyl) acétyl] décahydro 8-(1-pyrrolidinyl) quinoléine.**

On opère comme au stade C de l'exemple 1 à partir de 471 mg d'acide p-nitrophényl acétique, 422 mg de carbonyldiimidazole et 417 mg de produit préparé comme au stade B de l'exemple 1 en maintenant l'agitation pendant 3 heures. Après cristallisation du chlorhydrate dans l'éthanol, on obtient 606 mg de produit attendu. F = 249 ° C (décomposition).

| Analyse : $C_{21}H_{29}N_3O_3$, HCl : 407,944 | | | | |
|---|---|---|---|---|
| Calculé :<br>Trouvé : | C% 61,83<br>61,8 | H% 7,41<br>7,5 | N% 10,30<br>10,1 | Cl% 8,67<br>8,5 |

**Exemple 9 : Chlorhydrate de (4a alpha, 8 alpha, 8a alpha) (±) 1-[(3,4-diméthoxyphényl) acétyl] décahydro 8-(1-pyrrolidinyl) quinoléine.**

On opère comme à l'exemple 8 en utilisant 510 mg d'acide 3,4-diméthoxy phényl acétique en maintenant l'agitation pendant 20 heures. On obtient 571 mg de chlorhydrate attendu. F = 250 ° C (décomposition).

| Analyse : $C_{23}H_{34}N_2O_3$, HCl : 422,999 | | | | |
|---|---|---|---|---|
| Calculé :<br>Trouvé : | C% 65,31<br>65,3 | H% 8,34<br>8,4 | N% 6,62<br>6,4 | Cl% 8,38<br>8,4 |

**Exemple 10 : Chlorhydrate de (4a alpha, 8 alpha, 8a alpha) (±) 1-[(2,4-dichlorophényl) acétyl] décahydro 8-(1-pyrrolidinyl) quinoléine.**

On opère comme à l'exemple 9 en utilisant 533 mg d'acide 2,4-dichloro phényl acétique et en

maintenant l'agitation pendant 4 heures. On obtient le produit attendu. F > 260°.

| Analyse : $C_{21}H_{28}Cl_2N_2O$, HCl : 431,836 | | | | |
|---|---|---|---|---|
| Calculé :<br>Trouvé : | C% 58,41<br>58,7 | H% 6,77<br>6,8 | N% 6,48<br>6,5 | Cl% 24,63<br>24,6 |

**Exemple 11 : Chlorhydrate de (4a alpha, 8 alpha, 8a alpha) (±) 1-[(1-naphtalényl)acétyl] décahydro 8-(1-pyrrolidinyl) quinoléine.**

On opère comme à l'exemple 8 en utilisant 484 mg d'acide alpha-naphtyl acétique en maintenant l'agitation pendant 20 heures. On obtient 769 mg de produit attendu. F ≃ 262°C.

| Analyse : $C_{25}H_{32}N_2O$, HCl : 413,007 | | | | |
|---|---|---|---|---|
| Calculé :<br>Trouvé : | C% 72,70<br>72,9 | H% 8,05<br>8,2 | N% 6,78<br>6,8 | Cl% 8,58<br>8,7 |

**Exemple 12 : Chlorhydrate de (4a alpha, 8 alpha, 8a alpha) (±) 1-[2-(3,4-dichlorophényl) 1-oxopropyl] décahydro 8-(1-pyrrolidinyl) quinoléine (isomère A).**

On opère comme à l'exemple 8 en utilisant 482 mg d'acide alpha-méthyl 3,4-dichlorophényl acétique, dl, en maintenant l'agitation pendant 24 heures. On obtient 334 mg de chlorhydrate attendu.
F = 260°C (décomposition).

| Analyse : $C_{22}H_{30}Cl_2N_2O$, HCl : 445,863 | | | | |
|---|---|---|---|---|
| Calculé :<br>Trouvé : | C% 59,26<br>59,2 | H% 7,01<br>7,0 | N% 6,28<br>6,3 | Cl% 23,85<br>23,6 |

**Exemple 13 : Chlorhydrate de (4a alpha, 8 alpha, 8a alpha) (±) 1-[2-(3,4-dichlorophényl) 1-oxopropyl] décahydro 8-(1-pyrrolidinyl) quinoléine (isomère B).**

On agite 40 heures à température ambiante 570 mg d'acide alpha-méthyl 3,4-dichlorophényl acétique, 417 mg de produit préparé comme au stade B de l'exemple 1 dans 5 cm3 de chlorure de méthylène en présence de 20 mg de 4-diméthylaminopyridine et de 635 mg de dicyclohexylcarbodiimide. On filtre le dicyclohexylurée formée, et concentre à sec le filtrat sous pression réduite. On reprend le résidu dans 50 cm3 d'éther, lave avec une solution aqueuse saturée en bicarbonate de sodium, puis à l'eau et sèche. On élimine les solvants sous pression réduite, reprend le résidu dans l'éther et essore le produit cristallisé. On concentre à sec les liqueurs mères de cristallisation et obtient 905 mg de produit brut que l'on chromatographie sur silice (éluant : acétate d'éthyle à 2 % de triéthylamine). On obtient 378 mg d'isomère A et 303 mg d'isomère B sous forme de base. On dissout 287 mg de base (isomère B) dans 1 cm3 d'éther, filtre, rince à l'éther et à l'éthanol, ajoute au filtrat 0,5 cm3 d'une solution éthanolique d'acide chlorhydrique (5,75 N), concentre sous pression réduite jusqu'à un volume de 0,5 cm3 et ajoute 10 cm3 d'éther. On essore le produit cristallisé, le sèche à 70°C sous pression réduite. On recueille 146 mg de produit attendu.
F ≃ 254°C (décomposition).

| Analyse : $C_{22}H_{30}Cl_2N_2O$, HCl : 445,863 | | | | |
|---|---|---|---|---|
| Calculé :<br>Trouvé : | C% 59,26<br>59,3 | H% 7,01<br>7,0 | N% 6,28<br>6,3 | Cl% 23,85<br>23,7 |

**Exemple 14 : Chlorhydrate de (4a alpha, 8 alpha, 8a alpha) (±) 1-[(benzo [b] thièn-4-yl) acétyl] décahydro 8-(1-pyrrolidinyl) quinoléine.**

On opère comme à l'exemple 8 en utilisant 500 mg d'acide 4-thia naphtalène acétique en maintenant l'agitation pendant 6 heures. On obtient 655 mg du chlorhydrate attendu.
F > 260 ° C.

| Analyse : $C_{23}H_{30}N_2OS$, HCl : 419,032 | | | | | |
|---|---|---|---|---|---|
| Calculé :<br>Trouvé : | C% 65,93<br>65,8 | H% 7,46<br>7,6 | N% 6,68<br>6,6 | S% 7,65<br>7,3 | Cl% 8,46<br>8,7 |

**Exemple 15 : Fumarate de (4a alpha, 8 alpha, 8a alpha) (±) 1-[(1H-indol-3-yl) acétyl] décahydro 8-(1-pyrrolidinyl) quinoléine.**

On prépare le produit sous forme de base en opérant comme à l'exemple 8, en utilisant 455 mg d'acide 3-indole acétique et en maintenant sous agitation pendant 40 heures. On prépare le fumarate en opérant comme à l'exemple 4 et obtient après recristallisation dans le méthanol 233 mg de produit attendu.
F > 260 ° C.

| Analyse : $C_{23}H_{31}N_3O$, 1/2 $C_4H_4O_4$ : 423,560 | | | |
|---|---|---|---|
| Calculé :<br>Trouvé : | C% 70,89<br>70,8 | H% 7,85<br>8 | N% 9,92<br>9,8 |

**Exemple 16 : Fumarate de (4a alpha, 8 alpha, 8a alpha) (±) 1-(phényl acétyl) décahydro 8-(1-pyrrolidinyl) quinoléine.**

On opère comme indiqué à l'exemple 14 en utilisant 442 mg d'acide phényl acétique, 527 mg de carbonyldiimidazole et 521 mg du produit préparé à l'exemple 1 stade B et en maintenant l'agitation pendant 16 heures. On obtient 641 mg du fumarate attendu après cristallisation dans l'éthanol. F = 228 ° C.

| Analyse : $C_{21}H_{30}N_2O$, $C_4H_4O_4$ : 442,560 | | | |
|---|---|---|---|
| Calculé :<br>Trouvé : | C% 67,85<br>67,8 | H% 7,74<br>7,8 | N% 6,33<br>6,3 |

**Exemple 17 : Fumarate de (4a alpha, 8 alpha, 8a alpha) (±) 1-[(4-méthylphényl) acétyl] décahydro 8-(1-pyrrolidinyl) quinoléine.**

On opère comme à l'exemple 8 en utilisant 390 mg d'acide p-tolyl acétique, en maintenant l'agitation pendant 6 heures. Le produit obtenu sous forme de base est transformé en fumarate selon le mode opératoire indiqué à l'exemple 4. Après recristallisation dans l'isopropanol, on obtient 458 mg de produit attendu. F = 198 ° C.

| Analyse : $C_{22}H_{32}N_2O$, 1/5 $C_4H_4O_4$ : 514,624 | | | |
|---|---|---|---|
| Calculé :<br>Trouvé : | C% 65,35<br>65,1 | H% 7,44<br>7,5 | N% 5,4<br>5,3 |

**Exemple 18 : Fumarate de (4a alpha, 8 alpha, 8a alpha) (±) 1-[(4-pyridinyl) acétyl] décahydro 8-(1-pyrrolidinyl) quinoléine.**

On opère comme à l'exemple 8, en utilisant 452 mg d'acide 4-pyridyl acétique en maintenant l'agitation pendant 3 heures. Le produit obtenu sous forme de base est transformé en fumarate selon le mode opératoire indiqué à l'exemple 4. On obtient 373 mg du fumarate attendu. F = 232 ° C.

| Analyse : $Cl_{20}H_{29}N_3O$, 1/5 $C_4H_4O_4$ : 501,585 | | | |
|---|---|---|---|
| Calculé :<br>Trouvé : | C% 62,26<br>62,2 | H% 7,03<br>7,1 | N% 8,38<br>8,4 |

**Exemple 19 : Fumarate de (4a alpha, 8 alpha, 8a alpha) (±) 1-(2-thiényl acétyl) décahydro 8-(1-pyrrolidinyl) quinoléine.**

On opère comme à l'exemple 13 en utilisant 512 mg d'acide thiophène acétique, 521 mg de produit préparé comme au stade B de l'exemple 1, 816 mg de dicyclohexylcarbodiimide et 10 mg de diméthylaminopyridine.

Après 42 heures d'agitation à température ambiante, on obtient le produit sous forme de base que l'on transforme en fumarate selon le mode opératoire indiqué à l'exemple 4. On recueille 648 mg du fumarate acide attendu.
F = 252 ° C (décomposition).

| Analyse : $C_{19}H_{28}N_2OS$, $C_4H_4O_4$ : 448,585 | | | | |
|---|---|---|---|---|
| Calculé :<br>Trouvé : | C% 61,58<br>61,5 | H% 7,19<br>7,3 | N% 6,24<br>6,2 | S% 7,15<br>7,02 |

**Exemple 20 : Chlorhydrate de (4a alpha, 8 alpha, 8a alpha) (±) 1-(3,4,5-triméthoxybenzoyl) décahydro 8-(1-pyrrolidinyl) quinoléine.**

On fait réagir pendant 40 heures à température ambiante, 510 mg de chlorure de 3,4,5-triméthoxybenzoyle et 417 mg de produit obtenu au stade B de l'exemple 1, dans l'éther. On obtient le produit brut sous forme de base que l'on transforme en chlorhydrate comme indiqué à l'exemple 1. On obtient 319 mg de produit attendu. F ≃ 260 ° C.

| Analyse : $C_{23}H_{34}N_2O_4$, HCl : 438,999 | | | | |
|---|---|---|---|---|
| Calculé :<br>Trouvé : | C% 62,93<br>62,7 | H% 8,04<br>8,1 | N% 6,38<br>6,3 | Cl% 8,07<br>8,0 |

**Exemple 21 : Fumarate de (4a alpha, 8 alpha, 8a alpha) (±) 1-(4-bromobenzoyl) décahydro 8-(1-pyrrolidinyl) quinoléine.**

On opère comme à l'exemple 20 en utilisant 483 mg de chlorure de bromobenzoyle, en maintenant la réaction 22 heures à température ambiante. On obtient le produit brut sous forme de base que l'on transforme en fumarate selon le mode opératoire indiqué à l'exemple 4. On recueille 457 mg de produit attendu. F = 206 ° C (décomposition).

| Analyse : $C_{20}H_{27}BrN_2O$, $C_4H_4O_4$ : 507,434 | | | | |
|---|---|---|---|---|
| Calculé :<br>Trouvé : | C% 56,80<br>56,6 | H% 6,16<br>6,2 | N% 5,52<br>5,5 | Br% 15,74<br>15,6 |

**Exemple 22 : Fumarate de (4a alpha, 8 alpha, 8a alpha) (±) 1-(3,4-dichlorobenzoyl) décahydro 8-(1-**

**pyrrolidinyl) quinoléine.**

On opère comme à l'exemple 20 en utilisant 461 mg de chlorure de 3,4-dichlorobenzoyle, en maintenant la réaction pendant 20 heures à température ambiante. On obtient le produit brut sous forme de base que l'on transforme en fumarate selon le mode opératoire indiqué à l'exemple 4. On recueille 547 mg de produit attendu. F = 202 °C.

| Analyse : $C_{20}H_{26}Cl_2N_2O$, $C_4H_4O_4$ : 497,423 | | | | |
|---|---|---|---|---|
| Calculé :<br>Trouvé : | C% 57,95<br>57,7 | H% 6,08<br>6,2 | N% 5,63<br>5,6 | Cl% 14,25<br>14,1 |

**Exemple 23 : Chlorhydrate de (4a alpha, 8 alpha, 8a alpha) (±) 1-[(3,4-dichlorophényl) acétyl] décahydro 8-diméthylamino quinoléine.**

**Stade A :** N,N-diméthyl 5,6,7,8-tétrahydro 8-quinoléinamine.

On mélange sous agitation pendant 75 minutes 4,08 g de chlorhydrate de la 8-chloro 5,6,7,8-tétrahydro quinoléine préparé comme indiqué à la fin de l'exemple I dans 20 cm3 d'une solution aqueuse de diméthylamine à 40 %. On chauffe 1 heure à 65 °C ± 2 °C, laisse refroidir à température ambiante, sature le milieu réactionnel avec du chlorure de sodium et ajoute 0,3 cm3 d'hydroxyde de sodium 2N. On extrait à l'éther, lave à l'eau, sèche et élimine le solvant sous pression réduite. On obtient 3,35 g de produit attendu utilisé tel quel dans le stade suivant.

**Stade B** : (4a alpha, 8 alpha, 8a alpha) (±) N,N-diméthyl décahydro 8-quinoléinamine (isomère cis A) et (4a alpha, 8 béta, 8a alpha) (±) N,N-diméthyl décahydro 8-quinoléinamine (isomère cis B).

1) Réduction catalytique.

On introduit dans un appareil à hydrogéner 3,35 g de produit obtenu au stade A, 33 cm3 de méthanol, 3,3 cm3 d'acide chlorhydrique en présence de 0,37 g d'oxyde de platine à 80 %. On hydrogène pendant 7 heures à 22 °-24 °C à la pression de 1840 mbars. On filtre le catalyseur, rince, concentre à sec sous pression réduite et obtient 4,85 g de produits attendus.

2) Cristallisation du chlorhydrate de l'isomère cis A.

On reprend l'extrait sec dans 15 cm3 d'isopropanol, amorce la cristallisation, abandonne 1 heure à température ambiante, filtre, rince les cristaux à l'isopropanol et à l'éther, les sèche sous pression réduite à 50 °C et recueille après recristallisation dans l'éthanol 1,27 g de produit attendu sous forme de chlorhydrate. F > 260 °C.

3) Retour à la base.

On dissout 1,237 g du produit obtenu ci-dessus dans 5 cm3 d'eau, sature avec du chlorure de sodium, ajoute 2 cm3 d'hydroxyde de sodium 2N, extrait à l'éther, sèche et élimine le solvant sous pression réduite. On obtient 0,962 g de produit attendu sous forme de base.

4) Préparation de l'isomère "cis B".

On réunit les liqueurs mères de cristallisation du chlorhydrate de l'isomère cis A et les concentre à sec sous pression réduite. On reprend le résidu dans 10 cm3 d'eau, sature en chlorure de sodium, alcalinise à l'aide d'hydroxyde de sodium 2N, extrait à l'éther, sèche et élimine le solvant sous pression réduite. Après chromatographie sur silice (éluant : acétate d'éthyle-méthanol-triéthylamine 85-10-5), on recueille 384 mg de produit attendu.

**Stade C** : Chlorhydrate de (4a alpha, 8 alpha, 8a alpha) (±) 1-[(3,4-dichlorophényl) acétyl] décahydro 8-diméthylamino quinoléine.

On ajoute 1,160 g de dicyclohexylcarbodiimide dans une solution comprenant 855 g de l'isomère cis A sous forme de base préparé au stade B 3) et 1,160 g d'acide 3,4-dichlorophényl acétique dans 15 cm3 de chlorure de méthylène. On agite pendant 18 heures, élimine par filtration l'urée formée, concentre à sec le filtrat sous pression réduite et reprend le résidu dans 50 cm3 d'éther, lave avec une solution aqueuse saturée en bicarbonate de sodium, extrait à l'éther, sèche et élimine le solvant sous pression réduite. On recueille 2,5 g de produit sous forme de base que l'on dissout dans 15 cm3 d'éthanol, ajoute 2 cm3 d'une solution éthanolique d'acide chlorhydrique 5,75 N, laisse cristalliser pendant 1 heure, filtre et sèche les cristaux, les rince à l'éthanol puis à l'éther et sèche à 50 ° C sous pression réduite. On obtient 1,064 g de produit attendu. F ≃ 256 ° C.

| Analyse : $C_{19}H_{26}Cl_2N_2O$, HCl : 405,798 | | | | |
|---|---|---|---|---|
| Calculé :<br>Trouvé : | C% 56,24<br>56,1 | H% 6,70<br>6,7 | N% 6,90<br>6,8 | Cl% 26,21<br>25,8 |

**Exemple 24 : Oxalate de (4a alpha, 8 béta, 8a alpha) (±) 1-[(3,4-dichlorophényl) acétyl] décahydro 8-diméthylamino quinoléine.**

On opère comme au stade C de l'exemple 23 à partir de 844 mg de l'isomère cis B préparé au stade B 4) de l'exemple 23. On obtient 2,065 g de produit sous forme de base. On dissout 1,22 g de cette base et 0,7 g d'acide oxalique dihydraté dans 5 cm3 d'éthanol, filtre, rince à l'éthanol, ajoute au filtrat 30 cm3 d'éther, essore les cristaux, les rince avec un mélange éthanol-éther (1-3) puis à l'éther et les sèche sous pression réduite à 70 ° C. On obtient 1,132 g d'oxalate attendu. F = 159 ° C.

| Analyse : $C_{10}H_{26}Cl_2N_2O$ : 459,373 | | | | |
|---|---|---|---|---|
| Calculé :<br>Trouvé : | C% 54,90<br>54,8 | H% 6,14<br>6,0 | N% 6,10<br>6,9 | Cl% 15,44<br>15,2 |

**Exemple 25 : Chlorhydrate de (4a alpha, 8 alpha, 8a alpha) (±) 1-[(3,4-dichlorophényl) acétyl] décahydro 8-(1-pipéridinyl) quinoléine.**

**Stade A** : 8-(1-pipéridinyl) 5,6,7,8-tétrahydroquinoléine.

On introduit 3,9 cm3 de pipéridine dans une solution comprenant 2 g de chlorhydrate de 8-chloro 5,6,7,8-tétrahydroquinoléine dans 5 cm3 d'eau, agite 15 minutes, chauffe 2 heures et demie à 57 ° C ± 2 ° C, refroidit à température ambiante, sature le milieu en chlorure de sodium et extrait à l'éther. On élimine le solvant sous pression réduite et obtient 2,07 g de produit attendu.

**Stade B** : (4a alpha, 8 alpha, 8 alpha) (±) décahydro 8-(1-pipéridinyl) quinoléine (isomère cis A) ; (4a alpha, 8 béta, 8a alpha) (±) décahydro 8-(1-pipéridinyl) quinoléine (isomère cis B) et (4a alpha, 8 alpha, 8a béta) (±) décahydro 8-(1-pipéridinyl) quinoléine (isomère trans A).

On hydrogène pendant 6 heures à la pression de 1850 mbars et à température ambiante 1,974 g de produit obtenu au stade A dans 30 cm3 d'éthanol en présence de 3 cm3 d'acide chlorhydrique et 0,2 g d'oxyde de platine ; on filtre le catalyseur, rince et concentre à sec le filtrat sous pression réduite. On reprend le résidu par 10 cm3 d'eau, ajoute 12 cm3 de lessive de soude, extrait à l'acétate d'éthyle, sèche et élimine le solvant sous pression réduite. On obtient 2,04 g de produit brut que l'on chromatographie sur silice (éluant : acétate d'éthyle-méthanol-triéthylamine 85-10-5). On obtient 551 mg d'isomère cis A, 733 mg d'isomère cis B et 353 mg d'isomère trans A.

**Stade C :** Chlorhydrate de (4a alpha, 8 alpha, 8a alpha) (±) 1-[(3,4-dichlorophényl) acétyl] décahydro 8-(1-pipéridinyl) quinoléine.

On fait réagir pendant 6 heures, 479 mg d'acide 3,4-dichlorophényl acétique et 467 mg d'isomère cis A

préparé au stade B dans 7,2 cm3 de chlorure de méthylène en présence de 482 mg de dicyclohexylcarbodiimide.

On filtre la dicyclohexylurée formée, concentre à sec le filtrat sous pression réduite et reprend le résidu dans 30 cm3 d'acétate d'éthyle. On lave la phase organique avec une solution aqueuse saturée en bicarbonate de sodium puis à l'eau, sèche et élimine le solvant sous pression réduite.

On obtient 1,117 g de produit brut sous forme de base que l'on transforme en chlorhydrate selon le mode opératoire indiqué à l'exemple 1. On recueille 600 mg de produit attendu. F > 260 ° C.

| Analyse : $C_{22}H_{30}Cl_2N_2O$, HCl : 445,863 | | | | |
|---|---|---|---|---|
| Calculé :<br>Trouvé : | C% 59,26<br>59,4 | H% 7,01<br>7,2 | N% 6,28<br>6,2 | Cl% 23,85<br>24,0 |

**Exemple 26 : Fumarate de (4a alpha, 8 béta, 8a alpha) (±) 1-[(3,4-dichlorophényl) acétyl] décahydro 8-(1-pipéridinyl) quinoléine.**

On opère comme au stade C de l'exemple 25 en utilisant 550 mg d'acide 3,4-dichlorophényl acétique et 537 mg d'isomère cis B préparé au stade B de l'exemple 25 et en laissant réagir pendant 20 heures à température ambiante. On obtient 1,296 g de produit brut sous forme de base que l'on transforme en fumarate comme indiqué à l'exemple 4. On recueille 202 mg de produit attendu. F ≃ 227 ° C.

| Analyse : $C_{22}H_{30}Cl_2N_2O$, $C_4H_4O_4$ : 525,177 | | | | |
|---|---|---|---|---|
| Calculé :<br>Trouvé : | C% 59,43<br>59,3 | H% 6,52<br>6,8 | N% 5,33<br>5,1 | Cl% 13,49<br>13,5 |

**Exemple 27 : Bromure de (4a alpha, 8 alpha, 8a alpha) (±) 1-[1-[(3,4-dichlorophényl) acétyl] décahydro 8-quinolényl] 1-méthyl pyrrolidinium.**

On ajoute 6 g de bromure de méthyle à une solution comprenant 401 mg de produit de l'exemple 1 sous forme de base dans 6 cm3 de tétrahydrofuranne. On agite 24 heures à température ambiante, essore, rince le produit cristallisé au tétrahydrofuranne puis à l'éther et sèche sous pression réduite à 70 ° -80 ° C. On obtient 455 mg de produit attendu.
F ≃ 170 ° C.

| Analyse : $C_{22}H_{31}BrCl_2N_2O$ : 490,319 | | | | | |
|---|---|---|---|---|---|
| Calculé :<br>Trouvé : | C% 53,89<br>53,8 | H% 6,37<br>6,5 | N% 5,71<br>5,5 | Cl% 14,46<br>13,7 | Br% 16,3<br>14,9 |

**Exemple 28 :**

On a préparé des comprimés répondant à la formule suivante :

| | |
|---|---|
| - produit de l'exemple 1<br>- excipient q.s.p. | 200 mg<br>800 mg |
| (détail de l'excipient : lactose, talc, amidon, stéarate de magnésium). | |

**Exemple 29 :**

On a préparé un soluté injectable (voie intra-musculaire) répondant à la formule suivante :

| | |
|---|---|
| - produit de l'exemple 1<br>- solvant stérile q.s.p. | 50 mg<br>5 ml. |

**ETUDE PHARMACOLOGIQUE**

1) Liaison au récepteur opiacé K in vitro.

On utilise des culots membranaires conservés à -30 °C pendant environ 30 jours et préparés à partir de cervelets de cobayes.

Ces culots sont remis en suspension dans le tampon Tris pH 7,7. On répartit des fractions de 2 ml dans des tubes à hémolyse et ajoute de la 9-$^3$H-ethylkétocyclazocine 1nM et le produit à étudier. Le produit est d'abord testé à $5X10^{-6}$ M (en triple). Lorsque le produit testé déplace de plus de 50 % la radioactivité liée spécifiquement au récepteur, il est testé à nouveau selon une gamme de 7 doses afin de déterminer la dose qui inhibe de 50 % la radioactivité liée spécifiquement au récepteur. On détermine ainsi la concentration inhibitrice 50 %).

La liaison non spécifique est déterminée par addition de produit connu sous le nom U-50488 H à $10^{-5}$ M (en triple). On incube à 25 °C pendant 40 minutes, remet au bain-marie à 0 °C, 5 minutes, filtre sous vide, rince au tampon Tris pH 7,7 et compte la radioactivité en présence de scintillant Triton® .

Les résultats sont exprimés directement en concentration inhibitrice 50 % ($CI_{50}$), c'est-à-dire en concentration de produit étudié, exprimée en nM, nécessaire pour déplacer 50 % de la radioactivité spécifique fixée sur le récepteur étudié.

Résultats :

| Produit de l'exemple | $CI_{50}$ en nM |
|---|---|
| 1 | 2,7 |
| 6 | 5 |
| 7 | 9,5 |
| 8 | 17 |
| 10 | 12 |
| 11 | 6 |
| 12 | 5,4 |
| 14 | 4,1 |
| 22 | 7,4 |

2) Activité analgésique

- test de la plaque chaude.

Des souris femelles pesant 22 à 24 g sont placées une par une sur une plaque de cuivre maintenue à 56 °C : la réaction à la douleur se manifeste par le léchage des pattes avant de l'animal ; le temps de cette réaction est noté et l'on ne retient que les souris réagissant en moins de 8 secondes.

Les animaux sont répartis par groupes homogènes et traités par le produit à étudier administré par voie sous cutanée, un groupe ne recevant que le véhicule. Le temps de réaction à la douleur est de nouveau mesuré 30 à 60 minutes après le traitement. La dose active ou $DA_{100}$ est la dose qui augmente le temps de réaction de 100 %, 30 minutes après le traitement compte tenu des variations du temps de réaction des animaux témoins.

Pour le produit de l'exemple 1, la $DA_{100}$ est de 20 mg/kg.

- test des étirements.

Le test employé est basé sur le fait signalé par R. KOSTER et Coll., (Fed. Proc., 1959, 18 412) selon

l'injection intrapéritonéale d'acide acétique provoque, chez la souris, des mouvements répétés d'étirement et de torsion pouvant persister pendant plus de 6 heures. Les analgésiques préviennent ou diminuent ce syndrome qui peut être considéré comme l'extériorisation d'une douleur abdominale diffuse. On utilise une solution d'acide acétique à 1 % dans l'eau. La dose déclenchant le syndrome est dans ces conditions de 0,01 cm3/g, soit 100 mg/kg d'acide acétique.

Le produit étudié est administré par voie buccale une demi-heure avant l'injection d'acide acétique, les souris étant à jeun depuis la veille de l'expérience.

Les étirements sont observés et comptés pour chaque souris pendant une période d'observation de 15 minutes commençant aussitôt après l'injection d'acide acétique.

Les résultats sont exprimés au moyen de la $DA_{50}$, c'est-à-dire la dose qui permet d'obtenir une diminution de 50 % du nombre des étirements par rapport aux animaux témoins.

Résultats :

| Produit de l'exemple | $DA_{50}$ en mg/kg |
|---|---|
| 1 | 18 |
| 6 | 21 |
| 8 | 20 |

3) Action antiarythmique chez le rat.

On trachéotomise des rats mâles pesant 300-350 g anesthésiés par voie intrapéritonéale à l'aide de 1,20 g/kg d'uréthane et les soumet à une respiration artificielle (40-50 insufflations de 3 ml/minute).

On implante des aiguilles en sous cutané de manière à enregistrer l'électrocardiogramme des rats sur le signal en dérivation DII.

On administre les produits à tester par voie intraveineuse.

Cinq minutes après l'administration du produit, on perfuse la veine jugulaire des rats avec 10 µg/mn sous 0,2 ml d'une solution d'aconitine et on note le temps d'apparition des troubles du rythme cardiaque.

Les résultats sont exprimés en pourcentage d'allongement du temps d'apparition des troubles du rythme cardiaque par rapport aux témoins et en fonction de la dose du produit testé.

Les résultats figurant sur le tableau ci-après montrent que certains des produits de la présente demande sont doués de bonnes propriétés antiarythmiques.

| Produit de l'exemple | Dose mg/kg | Pourcentage d'allongement du temps |
|---|---|---|
| 1 | 10<br>5<br>2,5<br>1 | + 113,5<br>+ 56,5<br>+ 31,5<br>+ 17,5 |
| 2 | 2,5<br>1<br>0,5<br>0,25 | + 182<br>+ 93<br>+ 46<br>+ 26 |
| 3 | 10<br>5<br>2,5 | + 35,5<br>+ 36<br>+ 17 |
| 4 | 2,5<br>1<br>0,5 | + 112<br>+ 38,5<br>+ 10,5 |
| 20 | 1<br>0,5<br>0,25 | + 141<br>+ 85<br>+ 45 |

4) Test de l'anoxie asphyxique.

L'étude est réalisée sur des rats mâles (Charles River CD) (250-300 g), anesthésiés à l'éther, trachéotomisés, paralysés avec du chlorhydrate de d-tubocurarine 0,5 mg/kg IV, et soumis à respiration artificielle avec un mélange de 70 % de protoxyde d'azote et de 30 % d'oxygène. La température corporelle est maintenue à 37° C par un contrôleur automatique de température. On a implanté sur la boite crânienne et fixé avec du ciment dentaire, deux électrodes en argent-chlorure d'argent, dans les régions du cortex visuel et du cervelet pour enregistrer l'E.E.G. (Electro encéphalogramme). On a cathétérisé une artère carotide commune pour enregistrer la pression artérielle et la fréquence cardiaque. Les valeurs de $paO_2$, $paCO_2$ et de pH sont mesurées avant l'anoxie et la fréquence de la pompe respiratoire est réglée pour obtenir des valeurs normales.

L'anoxie est obtenue en déconnectant la pompe respiratoire selon la technique décrite par I. ROSNER, J. LEGROS et C. BERGER, Arch. Int. Pharmacodyn. 194, 375 (1971).

Après 3 minutes, la pompe respiratoire est remise en marche et la ventilation maintenue pendant 30 minutes.

Avant l'anoxie, à la fin de l'anoxie et 2, 10 et 30 minutes après la remise en marche de la ventilation, l'E.E.G. est enregistré sur un encéphalographe et l'analyse des spectres de puissance de l'E.E.G. est réalisée par un ordinateur digital PDP 11/34. Pendant l'enregistrement de l'E.E.G., des précautions sont prises pour éviter les stimulations visuelles et acoustiques.

On sélectionne 5 périodes de 10 secondes chaque minute, par contrôle visuel, afin d'exclure l'artéfact et l'analyse est faite par transformation de Fourier. Le spectre de puissance est évaluée entre 0 et 25 Hz, avec une résolution de 0,2 Hz.

Le produit à tester est dissous dans du Méthocel® à 0,5 % et administré par voie intraveineuse à 1 et 5 mg/kg, 3 minutes avant l'arrêt de la pompe.

Les valeurs de $paO_2$, $paCO_2$ et du pH sont de nouveau mesurées 30 minutes après l'anoxie. La pression artérielle moyenne (P.A.M.) et la fréquence cardiaque (F.C.) ont été enregistrées.

On a utilisé des groupes de 10 animaux.

Les résultats sont donnés dans le tableau 1.

La puissance totale et les énergies des différentes bandes de fréquence sont exprimées en pourcentages de celles enregistrées pendant le contrôle avant l'anoxie. Les E.S. (Ecart Standard) sont reportés uniquement pour indiquer la dispersion des données. Le test de Mann Whitney U est utilisé pour calculer le degré de signification des différences entre les témoins (sérum physiologique) et le groupe traité.
ns = $P > 0,05$ ; * = $P < 0,05$ ; ** = $P < 0,01$.

Les résultats obtenus avec le produit de l'exemple 1 sont reportés dans le tableau 1.

TABLEAU 1

Analyse spectrale de l'électroencéphalogramme de rats anesthésiés après 3 minutes d'anoxie asphyxique.

| Traitements | dose mg/kg i.v. | Avant | Fin de l'anoxie | Temps en minutes après l'anoxie 2 | 10 | 30 |
|---|---|---|---|---|---|---|
| | | | | DELTA | | |
| Témoins | - | 100 ± 0 | 1 ± 0 | 3 ± 1 | 902 ± 250 | 278 ± 44 |
| Produit de | 5 | 100 ± 0 | 1 ± 0 | 243 ± 68** | 127 ± 25** | 123 ± 14** |
| l'exemple 1 | 1 | 100 ± 0 | 1 ± 0 | 214 ± 60** | 404 ± 123 * | 225 ± 32 |
| | | | | THETA | | |
| Témoins | - | 100 ± 0 | 0 ± 0 | 1 ± 0 | 40 ± 7 | 71 ± 5 |
| Produit de | 5 | 100 ± 0 | 0 ± 0 | 6 ± 2** | 63 ± 11 | 94 ± 14 * |
| l'exemple 1 | 1 | 100 ± 0 | 0 ± 0 | 6 ± 2** | 57 ± 8 * | 72 ± 6 |
| | | | | ALPHA | | |
| Témoins | - | 100 ± 0 | 0 ± 0 | 1 ± 0 | 69 ± 10 | 83 ± 11 |
| Produit de | 5 | 100 ± 0 | 0 ± 0 | 25 ± 5** | 55 ± 4 | 80 ± 8 |
| l'exemple 1 | 1 | 100 ± 0 | 0 ± 0 | 19 ± 6** | 73 ± 5 | 79 ± 6 |
| | | | | BETA | | |
| Témoins | - | 100 ± 0 | 0 ± 0 | 1 ± 0 | 90 ± 11 | 90 ± 10 |
| Produit de | 5 | 100 ± 0 | 0 ± 0 | 33 ± 5** | 103 ± 14 | 102 ± 9 |
| l'exemple 1 | 1 | 100 ± 0 | 0 ± 0 | 21 ± 7** | 96 ± 11 | 87 ± 5 |
| | | | | PUISSANCE TOTALE | | |
| Témoins | - | 100 ± 0 | 0 ± 0 | 1 ± 0 | 252 ± 76 | 117 ± 9 |
| Produit de | 5 | 100 ± 0 | 0 ± 0 | 68 ± 14** | 79 ± 6** | 100 ± 8 |
| l'exemple 1 | 1 | 100 ± 0 | 0 ± 0 | 70 ± 22** | 173 ± 41 | 120 ± 9 |

* = P < 0,05    ** = P < 0,001 (Test de Mann-Whitney U)

Résultats :

Le produit administré par voie intraveineuse à la dose de 5 mg/kg, provoque une remarquable anticipation de la récupération de l'activité électrocorticale sur toutes les bandes de fréquences.

Trente minutes après l'anoxie, les valeurs des différentes bandes de fréquence du groupe traité avec 5 mg/kg sont presque égales aux valeurs de base, tandis que dans les contrôles, il persiste une importante composante lente (bande delta) qui indique un état de souffrance cérébrale encore présent.

Même à la dose d'1 mg/kg, on observe une anticipation de la récupération de l'activité électrocorticale, tandis que l'effet sur la normalisation du tracé est moins évident.

En effet après 30 minutes de la fin de l'anoxie, la valeur de la bande delta est encore environ le double de la valeur de base.

## Revendications

### Revendications pour les Etats contractants suivants : BE, CH, DE, FR, GB, IT, LI, LU, NL, SE

**1.** Composés de formule (I) :

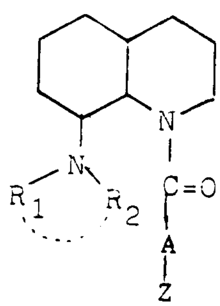

(I)

dans laquelle $R_1$ et $R_2$, identiques ou différents, représentent un atome d'hydrogène ou un radical alcoyle renfermant de 1 à 5 atomes de carbone ou $R_1$ et $R_2$ forment ensemble avec l'atome d'azote auquel ils sont liés un radical pyrrolidinyle, pipérazinyle, pipéridinyle ou morpholinyle, ces radicaux étant éventuellement substitués par un radical alcoyle ou alcoxy renfermant de 1 à 5 atomes de carbone, A représente une chaîne $(CH_2)_n$ dans laquelle n représente un nombre de 0 à 5, ou A représente une chaîne alcoylène substituée par un radical alcoyle renfermant au total de 2 à 8 atomes de carbone, Z représente un radical phényle éventuellement substitué par un ou plusieurs radicaux identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z représente un radical naphtyle, un radical indényle, pyridinyle, thiényle, thiazolyle, oxazolyle, isoxazolyle, imidazolyle, indolyle, quinolyle, benzofuranyle, benzo [b] thiényle, benzimidazolyle, benzoxazolyle ou benzothiazolyle tous ces radicaux étant éventuellement substitués par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes, lesdits composés de formule (I) pouvant être dans toutes les formes énantioméres et diastéréoisomères possibles et sous forme de sels d'addition avec les acides ou de sels d'ammonium quaternaire.

**2.** Composés de formule (I) telle que définie à la revendication 1, dans laquelle $R_1$ et $R_2$ représentent un radical méthyle ou éthyle, ou forment ensemble avec l'atome d'azote auquel ils sont liés, un radical pyrrolidinyle ou pipéridinyle, A représente une chaîne $(CH_2)_n$ où n est égal à 0 ou 1, ou une chaîne 1,1-éthanediyl, Z représente un radical phényle, éventuellement substitué par un ou plusieurs radicaux identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z représente un radical naphtyle, indényle, pyridinyle, thiényle, thiazolyle, oxazolyle, isoxazolyle, imidazolyle, indolyle, quinolyle, benzofuranyle, benzo [b] thiényle, benzimidazolyle, benzoxazolyle ou benzothiazolyle tous ces radicaux étant éventuellement substitués par un ou plusieurs substituants identiques ou différents, choisis dans le

groupe constitué par les radicaux alcoyles renfermant de 1 à 5 atomes de carbone, les radicaux alcoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes ainsi que leurs sels d'addition avec les acides et leurs sels d'ammonium quaternaire.

**3.** Composés de formule (I) telle que définie à la revendication 2, dans laquelle $R_1$, $R_2$ et A ont la signification déjà indiquée et Z représente un radical phényle, éventuellement substitué par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z représente un radical naphtyle, pyridinyle, thiényle, indolyle, benzo [b] thiényle, tous ces radicaux étant éventuellement substitués par un ou plusieurs substituants identiques ou différents, choisis dans le groupe constitué par les radicaux alcoyles renfermant de 1 à 5 atomes de carbone, les radicaux alkoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes ainsi que leurs sels d'addition avec les acides et leurs sels d'ammonium quaternaire.

**4.** Composés de formule (I) telle que définie à l'une quelconque des revendications 1 à 3, dans laquelle $R_1$ et $R_2$ forment ensemble avec l'atome d'azote auquel ils sont liés, un radical pyrrolidinyle, A représente une chaîne $(CH_2)_n$ où n est égal à 0 ou 1, ou une chaîne 1,1-éthanediyl, Z représente un radical phényle substitué par un ou plusieurs substituants choisis dans le groupe constitué par les atomes d'halogènes et le radical trifluorométhyle ou Z représente un radical naphtyle ou benzo [b] thiényle, ainsi que leurs sels d'addition avec les acides et leurs sels d'ammonium quaternaire.

**5.** L'un quelconque des composés de formule (I) telle que définie à la revendication 1, dont les noms suivent :

- la (4a alpha, 8 alpha, 8a alpha) (±) décahydro 1-[(3,4-dichlorophényl) acétyl] 8-(1-pyrrolidinyl) quinoléine,
- la (4a alpha, 8 alpha, 8a alpha) (±) décahydro 1-[[4-(trifluorométhyl) phényl] acétyl] 8-(1-pyrrolidinyl) quinoléine,
- la (4a alpha, 8 alpha, 8a alpha) (±) décahydro 1-[(4-bromophényl) acétyl] 8-(1-pyrrolidinyl) quinoléine,
- la (4a alpha, 8 alpha, 8a alpha) (±) décahydro 1-[2-(3,4-dichloro-phényl) 1-oxopropyl] 8-(1-pyrrolidinyl) quinoléine,
- la (4a alpha, 8 alpha, 8a alpha) (±) décahydro 1-(3,4-dichlorobenzoyl) 8-(1-pyrrolidinyl) quinoléine,
- la (4a alpha, 8 alpha, 8a alpha) (±) décahydro 1-[(4-benzo [b] thiényl) acétyl) 8-(1-pyrrolidinyl) quinoléine,
- la (4a alpha, 8 alpha, 8a alpha) (±) décahydro 1-[(1-naphtalényl) acétyl] 8-(1-pyrrolidinyl) quinoléine,

ainsi que leurs sels d'addition avec les acides et leurs sels d'ammonium quaternaire.

**6.** Procédé de préparation des composés de formule (I) tels que définis aux revendications 1 à 3, caractérisé en ce que l'on condense la 8-chloro 5,6,7,8-tétrahydro quinoléine, de formule :

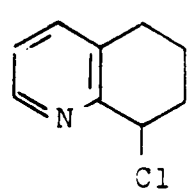

avec une amine de formule :

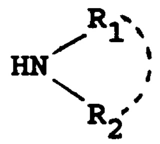

dans laquelle $R_1$ et $R_2$ ont les significations indiquées précédemment pour obtenir un composé de formule II :

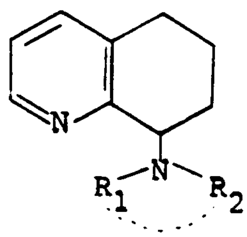

(II)

que l'on réduit pour obtenir un composé de formule III :

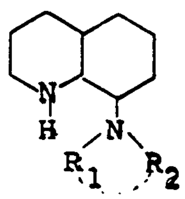

(III)

que l'on condense avec un composé de formule IV ou un dérivé fonctionnel de ce composé :

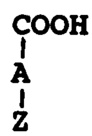

(IV)

dans laquelle A et Z ont les significations indiquées précédemment pour obtenir un composé de formule (I) sous toutes les formes énantiomères et diastéréoisomères possibles, que l'on traite, si désiré, avec un acide minéral ou organique pour obtenir un sel ou avec un halogénure d'alcoyle pour obtenir un sel d'ammonium quaternaire.

**7.** Procédé de préparation des composés de formule (I) tel que défini à la revendication 6, caractérisé en ce que l'on effectue la réduction du composé de formule II par un agent de réduction chimique et l'on obtient préférentiellement des composés de formule (I) dans laquelle la jonction du cycle est trans et en ce que l'on effectue la réduction du composé de formule II par hydrogénation catalytique et l'on obtient préférentiellement des composés de formule (I) dans laquelle la jonction du cycle est cis.

**8.** A titre de médicaments, les produits de formule (I) tels que définis aux revendications 1 à 4.

**9.** A titre de médicaments, les produits de la revendication 5.

**10.** Les compositions pharmaceutiques contenant à titre de principe actif, l'un au moins des médicaments, tels que définis aux revendications 8 et 9.

**11.** A titre de produit industriel nouveau, la 8-chloro 5,6,7,8-tétrahydroquinoléine.

**12.** A titre de produits industriels nouveaux, les produits de formules II et III telles que définies à la revendication 6.

**Revendications pour l'Etat contractant suivant : AT**

**1.** Procédé de préparation des composés de formule (I) :

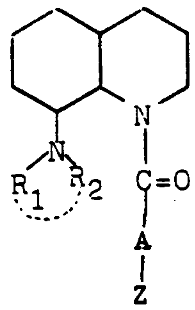

(I)

dans laquelle $R_1$ et $R_2$, identiques ou différents, représentent un atome d'hydrogène ou un radical alcoyle renfermant de 1 à 5 atomes de carbone ou $R_1$ et $R_2$ forment ensemble avec l'atome d'azote auquel ils sont liés un radical pyrrolidinyle, pipérazinyle, pipéridinyle ou morpholinyle, ces radicaux étant éventuellement substitués par un radical alcoyle ou alcoxy renfermant de 1 à 5 atomes de carbone, A représente une chaîne $(CH_2)_n$ dans laquelle n représente un nombre de 0 à 5, ou A représente une chaîne alcoylène substituée par un radical alcoyle renfermant au total de 2 à 8 atomes de carbone, Z représente un radical phényle éventuellement substitué par un ou plusieurs radicaux identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z représente un radical naphtyle, un radical indényle, pyridinyle, thiényle, thiazolyle, oxazolyle, isoxazolyle, imidazolyle, indolyle, quinolyle, benzofuranyle, benzo [b] thiényle, benzimidazolyle, benzoxazolyle ou benzothiazolyle tous ces radicaux étant éventuellement substitués par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes, lesdits composés de formule (I) pouvant être dans toutes les formes énantioméres et diastéréoisomères possibles et sous forme de sels d'addition avec les acides ou de sels d'ammonium quaternaire, caractérisé en ce que l'on condense la 8-chloro 5,6,7,8-tétrahydro quinoléine, de formule :

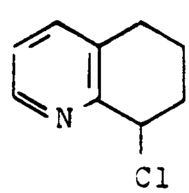

avec une amine de formule :

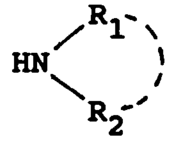

dans laquelle $R_1$ et $R_2$ ont les significations indiquées précédemment pour obtenir un composé de

formule II :

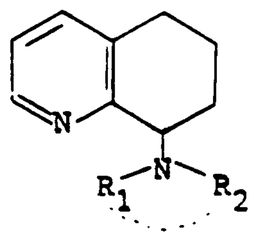

(II)

que l'on réduit pour obtenir un composé de formule III :

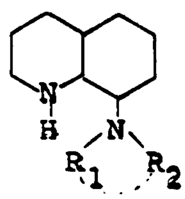

(III)

que l'on condense avec un composé de formule IV ou un dérivé fonctionnel de ce composé :

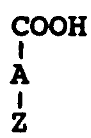

(IV)

dans laquelle A et Z ont les significations indiquées précédemment pour obtenir un composé de formule (I) sous toutes les formes énantiomères et diastéréoisomères possibles, que l'on traite, si désiré, avec un acide minéral ou organique pour obtenir un sel ou avec un halogénure d'alcoyle pour obtenir un sel d'ammonium quaternaire.

2. Procédé de préparation des composés de formule (I) tel que défini à la revendication 1, caractérisé en ce que l'on effectue la réduction du composé de formule II par un agent de réduction chimique et l'on obtient préférentiellement des composés de formule (I) dans laquelle la jonction du cycle est trans et en ce que l'on effectue la réduction du composé de formule II par hydrogénation catalytique et l'on obtient préférentiellement des composés de formule (I) dans laquelle la jonction du cycle est cis.

3. Procédé selon la revendication 1, caractérisé en ce que l'on utilise au départ une amine de formule

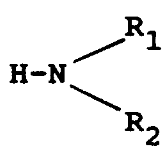

dans laquelle $R_1$ et $R_2$ représentent un radical méthyle ou éthyle, ou forment ensemble avec l'atome d'azote auquel ils sont liés, un radical pyrrolidinyle ou pipéridinyle, et un composé de formule (IV) ou un dérivé fonctionnel de ce composé, formule (IV) dans laquelle A représente une chaîne $(CH_2)_n$ où n est égal à 0 ou 1, ou une chaîne 1,1-éthanediyl, Z représente un radical phényle éventuellement substitué par un ou plusieurs radicaux identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z

représente un radical naphtyle, un radical indényle, pyridinyle, thiényle, thiazolyle, oxazolyle, isoxazolyle, imidazolyle, indolyle, quinolyle, benzofuranyle, benzo [b] thiényle, benzimidazolyle, benzoxazolyle ou benzothiazolyle tous ces radicaux étant éventuellement substitués par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes.

**4.** Procédé selon la revendication 3, caractérisé en ce que l'on utilise au départ un composé de formule (IV) ou un dérivé fonctionnel de ce composé, formule (IV) dans laquelle Z représente un radical phényle, éventuellement substitué par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z représente un radical naphtyle, pyridinyle, thiényle, indolyle, benzo [b] thiényle, tous ces radicaux étant éventuellement substitués par un ou plusieurs substituants identiques ou différents, choisis dans le groupe constitué par les radicaux alcoyles renfermant de 1 à 5 atomes de carbone, les radicaux alkoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes.

**5.** Procédé selon l'une quelconque des revendications 1, 3 ou 3, caractérisé en ce que l'on utilise au départ une amine de formule :

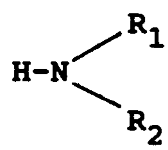

dans laquelle $R_1$ et $R_2$ forment ensemble avec l'atome d'azote auquel ils sont liés, un radical pyrrolidinyle et un composé de formule (IV) ou un dérivé fonctionnel de ce composé, formule (IV) dans laquelle A représente une chaîne $(CH_2)_n$ où n est égal à 0 ou 1, ou une chaîne 1,1-éthanediyl, Z représente un radical phényle substitué par un ou plusieurs substituants choisis dans le groupe constitué par les atomes d'halogènes et le radical trifluorométhyle ou Z représente un radical naphtyle ou benzo [b] thiényle.

**6.** Procédé selon la revendication 5, pour la préparation de la (4a alpha, 8 alpha, 8a alpha) (±) décahydro 1-[(3,4-dichlorophényl) acétyl] 8-(1-pyrrolidinyl) quinoléine et de ses sels d'addition avec les acides, caractérisé en ce que l'on utilise au départ une amine de formule

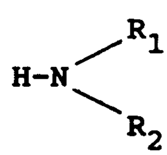

dans laquelle $R_1$ et $R_2$ forment ensemble avec l'atome d'azote auquel ils sont liés, un radical pyrrolidinyle et un composé de formule (IV) ou un dérivé fonctionnel de ce composé, formule (IV) dans laquelle A représente une chaîne $-CH_2-$ et Z représente un radical 3,4-dichloro-phényl.

**7.** Procédé selon la revendication 5, pour la préparation des composés de formule (I) telle que définie à la revendication 1, dont les noms suivent :

- la (4a alpha, 8 alpha, 8a alpha) (±) décahydro 1-[[4- (trifluorométhyl) phényl] acétyl] 8-(1-pyrrolidinyl) quinoléine,

- la (4a alpha, 8 alpha, 8a alpha) (±) décahydro 1-[(4-bromophényl) acétyl] 8-(1-pyrrolidinyl) quinoléine,
- la (4a alpha, 8 alpha, 8a alpha) (±) décahydro 1-[2-(3,4-dichloro-phényl) 1-oxopropyl] 8-(1-pyrrolidinyl) quinoléine,
- la (4a alpha, 8 alpha, 8a alpha) (±) décahydro 1-(3,4-dichlorobenzoyl) 8-(1-pyrrolidinyl) quinoléine,
- la (4a alpha, 8 alpha, 8a alpha) (±) décahydro 1-[(4-benzo [b] thiényl) acétyl] 8-(1-pyrrolidinyl) quinoléine,
- la (4a alpha, 8 alpha, 8a alpha) (±) décahydro 1-[(1-naphtalényl) acétyl] 8-(1-pyrrolidinyl) quinoléine,

ainsi que leurs sels d'addition avec les acides et leurs sels d'ammonium quaternaire, caractérisé en ce que l'on utilise au départ une amine de formule

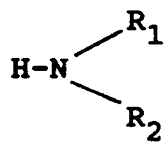

dans laquelle $R_1$ et $R_2$ forment ensemble avec l'atome d'azote auquel ils sont liés, un radical pyrrolidinyle et un composé de formule (IV) ou un dérivé fonctionnel de ce composé, formule (IV) dans laquelle A et Z ont les valeurs correspondant aux composés nommés plus haut.

**Revendications pour l'Etat contractant suivant : ES**

**1.** Procédé de préparation des composés de formule (I) :

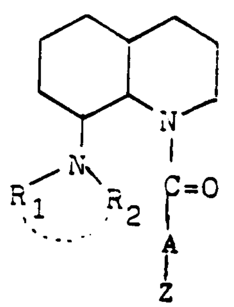

(I)

dans laquelle $R_1$ et $R_2$, identiques ou différents, représentent un atome d'hydrogène ou un radical alcoyle renfermant de 1 à 5 atomes de carbone ou $R_1$ et $R_2$ forment ensemble avec l'atome d'azote auquel ils sont liés un radical pyrrolidinyle, pipérazinyle, pipéridinyle ou morpholinyle, ces radicaux étant éventuellement substitués par un radical alcoyle ou alcoxy renfermant de 1 à 5 atomes de carbone, A représente une chaîne $(CH_2)_n$ dans laquelle n représente un nombre de 0 à 5, ou A représente une chaine alcoylène substituée par un radical alcoyle renfermant au total de 2 à 8 atomes de carbone, Z représente un radical phényle éventuellement substitué par un ou plusieurs radicaux identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z représente un radical naphtyle, un radical indényle, pyridinyle, thiényle, thiazolyle, oxazolyle, isoxazolyle, imidazolyle, indolyle, quinolyle, benzofuranyle, benzo [b] thiényle, benzimidazolyle, benzoxazolyle ou benzothiazolyle tous ces radicaux étant éventuellement substitués par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes, lesdits composés de formule (I) pouvant

être dans toutes les formes énantioméres et diastéréoisomères possibles et sous forme de sels d'addition avec les acides ou de sels d'ammonium quaternaire, caractérisé en ce que l'on condense la 8-chloro 5,6,7,8-tétrahydro quinoléine, de formule :

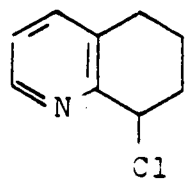

avec une amine de formule :

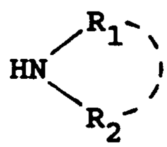

dans laquelle $R_1$ et $R_2$ ont les significations indiquées précédemment pour obtenir un composé de formule II :

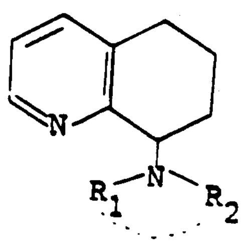

(II)

que l'on réduit pour obtenir un composé de formule III :

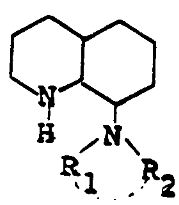

(III)

que l'on condense avec un composé de formule IV ou un dérivé fonctionnel de ce composé :

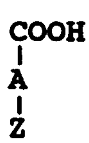

(IV)

dans laquelle A et Z ont les significations indiquées précédemment pour obtenir un composé de formule (I) sous toutes les formes énantiomères et diastéréoisomères possibles, que l'on traite, si désiré, avec un acide minéral ou organique pour obtenir un sel ou avec un halogénure d'alcoyle pour obtenir un sel d'ammonium quaternaire.

**2.** Procédé de préparation des composés de formule (I) tel que défini à la revendication 1, caractérisé en ce que l'on effectue la réduction du composé de formule II par un agent de réduction chimique et l'on obtient préférentiellement des composés de formule (I) dans laquelle la jonction du cycle est trans et en ce que l'on effectue la réduction du composé de formule II par hydrogénation catalytique et l'on obtient préférentiellement des composés de formule (I) dans laquelle la jonction du cycle est cis.

**3.** Procédé selon la revendication 1, caractérisé en ce que l'on utilise au départ une amine de formule

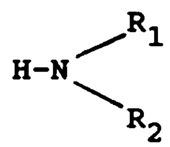

dans laquelle $R_1$ et $R_2$ représentent un radical méthyle ou éthyle, ou forment ensemble avec l'atome d'azote auquel ils sont liés, un radical pyrrolidinyle ou pipéridinyle, et un composé de formule (IV) ou un dérivé fonctionnel de ce composé, formule (IV) dans laquelle A représente une chaîne $(CH_2)_n$ où n est égal à 0 ou 1, ou une chaîne 1,1-éthanediyl, Z représente un radical phényle, éventuellement substitué par un ou plusieurs radicaux identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z représente un radical naphtyle, un radical indényle, pyridinyle, thiényle, thiazolyle, oxazolyle, isoxazolyle, imidazolyle, indolyle, quinolyle, benzofuranyle, benzo [b] thiényle, benzimidazolyle, benzoxazolyle ou benzothiazolyle tous ces radicaux étant éventuellement substitués par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes.

**4.** Procédé selon la revendication 3, caractérisé en ce que l'on utilise au départ un composé de formule (IV) ou un dérivé fonctionnel de ce composé, formule (IV) dans laquelle Z représente un radical phényle, éventuellement substitué par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z représente un radical naphtyle, pyridinyle, thiényle, indolyle, benzo [b] thiényle, tous ces radicaux étant éventuellement substitués par un ou plusieurs substituants identiques ou différents, choisis dans le groupe constitué par les radicaux alcoyles renfermant de 1 à 5 atomes de carbone, les radicaux alkoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes.

**5.** Procédé selon l'une quelconque des revendications 1, 3 ou 4, caractérisé en ce que l'on utilise au départ une amine de formule :

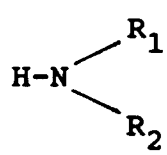

dans laquelle $R_1$ et $R_2$ forment ensemble avec l'atome d'azote auquel ils sont liés, un radical pyrrolidinyle et un composé de formule (IV) ou un dérivé fonctionnel de ce composé, formule (IV) dans

laquelle A représente une chaîne $(CH_2)_n$ où n est égal à 0 ou 1, ou une chaîne 1,1-éthanediyl, Z représente un radical phényle substitué par un ou plusieurs substituants choisis dans le groupe constitué par les atomes d'halogènes et le radical trifluorométhyle ou Z représente un radical naphtyle ou benzo [b] thiényle.

**6.** Procédé selon la revendication 5, pour la préparation de la (4a alpha, 8 alpha, 8a alpha) (±) décahydro 1-[(3,4-dichlorophényl) acétyl] 8-(1-pyrrolidinyl) quinoléine et de ses sels d'addition avec les acides, caractérisé en ce que l'on utilise au départ une amine de formule

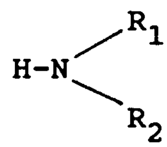

dans laquelle $R_1$ et $R_2$ forment ensemble avec l'atome d'azote auquel ils sont liés, un radical pyrrolidinyle et un composé de formule (IV) ou un dérivé fonctionnel de ce composé, formule (IV) dans laquelle A représente une chaîne $-CH_2-$ et Z représente un radical 3,4-dichloro-phényl.

**7.** Procédé selon la revendication 5, pour la préparation des composés de formule (I) telle que définie à la revendication 1, dont les noms suivent :

- la (4a alpha, 8 alpha, 8a alpha) (±) décahydro 1-[[4-(trifluorométhyl) phényl] acétyl] 8-(1-pyrrolidinyl) quinoléine,
- la (4a alpha, 8 alpha, 8a alpha) (±) décahydro 1-[(4-bromophényl) acétyl] 8-(1-pyrrolidinyl) quinoléine,
- la (4a alpha, 8 alpha, 8a alpha) (±) décahydro 1-[2-(3,4-dichloro-phényl) 1-oxopropyl] 8-(1-pyrrolidinyl) quinoléine,
- la (4a alpha, 8 alpha, 8a alpha) (±) décahydro 1-(3,4-dichlorobenzoyl) 8-(1-pyrrolidinyl) quinoléine,
- la (4a alpha, 8 alpha, 8a alpha) (±) décahydro 1-[(4-benzo [b] thiényl) acétyl] 8-(1-pyrrolidinyl) quinoléine,
- la (4a alpha, 8 alpha, 8a alpha) (±) décahydro 1-[(1-naphtalényl) acétyl] 8-(1-pyrrolidinyl) quinoléine,

ainsi que leurs sels d'addition avec les acides et leurs sels d'ammonium quaternaire, caractérisé en ce que l'on utilise au départ une amine de formule

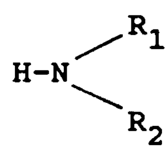

dans laquelle $R_1$ et $R_2$ forment ensemble avec l'atome d'azote auquel ils sont liés, un radical pyrrolidinyle et un composé de formule (IV) ou un dérivé fonctionnel de ce composé, formule (IV) dans laquelle A et Z ont les valeurs correspondant aux composés nommés plus haut.

**Revendications pour l'Etat contractant suivant : GR**

**1.** Procédé de préparation des composés de formule (I) :

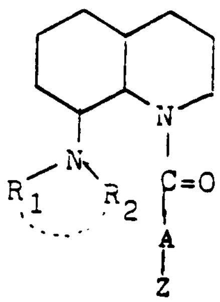

(I)

dans laquelle $R_1$ et $R_2$, identiques ou différents, représentent un atome d'hydrogène ou un radical alcoyle renfermant de 1 à 5 atomes de carbone ou $R_1$ et $R_2$ forment ensemble avec l'atome d'azote auquel ils sont liés un radical pyrrolidinyle, pipérazinyle, pipéridinyle ou morpholinyle, ces radicaux étant éventuellement substitués par un radical alcoyle ou alcoxy renfermant de 1 à 5 atomes de carbone, A représente une chaîne $(CH_2)_n$ dans laquelle n représente un nombre de 0 à 5, ou A représente une chaîne alcoylène substituée par un radical alcoyle renfermant au total de 2 à 8 atomes de carbone, Z représente un radical phényle éventuellement substitué par un ou plusieurs radicaux identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z représente un radical naphtyle, un radical indényle, pyridinyle, thiényle, thiazolyle, oxazolyle, isoxazolyle, imidazolyle, indolyle, quinolyle, benzofuranyle, benzo [b] thiényle, benzimidazolyle, benzoxazolyle ou benzothiazolyle tous ces radicaux étant éventuellement substitués par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes, lesdits composés de formule (I) pouvant être dans toutes les formes énantioméres et diastéréoisomères possibles et sous forme de sels d'addition avec les acides ou de sels d'ammonium quaternaire, caractérisé en ce que l'on condense la 8-chloro 5,6,7,8-tétrahydro quinoléine, de formule :

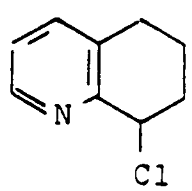

avec une amine de formule :

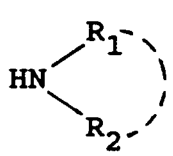

dans laquelle $R_1$ et $R_2$ ont les significations indiquées précédemment pour obtenir un composé de formule II :

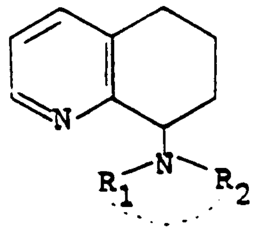

(II)

que l'on réduit pour obtenir un composé de formule III :

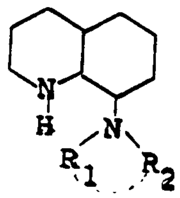

(III)

que l'on condense avec un composé de formule IV ou un dérivé fonctionnel de ce composé :

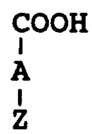

(IV)

dans laquelle A et Z ont les significations indiquées précédemment pour obtenir un composé de formule (I) sous toutes les formes énantiomères et diastéréoisomères possibles, que l'on traite, si désiré, avec un acide minéral ou organique pour obtenir un sel ou avec un halogénure d'alcoyle pour obtenir un sel d'ammonium quaternaire.

**2.** Procédé de préparation des composés de formule (I) tel que défini à la revendication 1, caractérisé en ce que l'on effectue la réduction du composé de formule II par un agent de réduction chimique et l'on obtient préférentiellement des composés de formule (I) dans laquelle la jonction du cycle est trans et en ce que l'on effectue la réduction du composé de formule II par hydrogénation catalytique et l'on obtient préférentiellement des composés de formule (I) dans laquelle la jonction du cycle est cis.

**3.** Procédé selon la revendication 1, caractérisé en ce que l'on utilise au départ une amine de formule

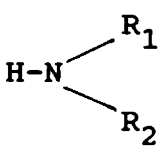

dans laquelle $R_1$ et $R_2$ représentent un radical méthyle ou éthyle, ou forment ensemble avec l'atome d'azote auquel ils sont liés, un radical pyrrolidinyle ou pipéridinyle, et un composé de formule (IV) ou un dérivé fonctionnel de ce composé, formule (IV) dans laquelle A représente une chaîne $(CH_2)_n$ où n est égal à 0 ou 1, ou une chaîne 1,1-éthanediyl, Z représente un radical phényle, éventuellement substitué par un ou plusieurs radicaux identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z

représente un radical naphtyle, un radical indényle, pyridinyle, thiényle, thiazolyle, oxazolyle, isoxazolyle, imidazolyle, indolyle, quinolyle, benzofuranyle, benzo [b] thiényle, benzimidazolyle, benzoxazolyle ou benzothiazolyle tous ces radicaux étant éventuellement substitués par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes.

**4.** Procédé selon la revendication 3, caractérisé en ce que l'on utilise au départ un composé de formule (IV) ou un dérivé fonctionnel de ce composé, formule (IV) dans laquelle Z représente un radical phényle, éventuellement substitué par un ou plusieurs substituants identiques ou différents, choisis dans le groupe constitué par les radicaux alcoyles renfermant de 1 à 5 atomes de carbone, les radicaux alcoxy renfermant de 1 à 5 atomes de carbone, les atomes d'halogènes, les radicaux hydroxyle, trifluorométhyle, nitro, amino, monoalkyl ou dialkylamino dont les radicaux alcoyles renferment de 1 à 5 atomes de carbone, soit Z représente un radical naphtyle, pyridinyle, thiényle, indolyle, benzo [b] thiényle, tous ces radicaux étant éventuellement substitués par un ou plusieurs substituants identiques ou différents, choisis dans le groupe constitué par les radicaux alcoyles renfermant de 1 à 5 atomes de carbone, les radicaux alkoxy renfermant de 1 à 5 atomes de carbone, les radicaux trifluorométhyle, nitro, amino, monoalkyl ou dialkylamino dont les radicaux alcoyles renferment de 1 à 5 atomes de carbone et phényle éventuellement substitué par un ou plusieurs radicaux alcoyle renfermant de 1 à 5 atomes de carbone, alcoxy renfermant de 1 à 5 atomes de carbone ou halogènes.

**5.** Procédé selon l'une quelconque des revendications 1, 3 ou 4, caractérisé en ce que l'on utilise au départ une amine de formule :

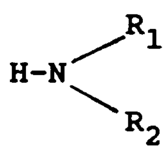

dans laquelle $R_1$ et $R_2$ forment ensemble avec l'atome d'azote auquel ils sont liés, un radical pyrrolidinyle et un composé de formule (IV) ou un dérivé fonctionnel de ce composé, formule (IV) dans laquelle A représente une chaîne $(CH_2)_n$ où n est égal à 0 ou 1, ou une chaîne 1,1-éthanediyl, Z représente un radical phényle substitué par un ou plusieurs substituants choisis dans le groupe constitué par les atomes d'halogènes et le radical trifluorométhyle ou Z représente un radical naphtyle ou benzo [b] thiényle.

**6.** Procédé selon la revendication 5, pour la préparation de la (4a alpha, 8 alpha, 8a alpha) (±) décahydro 1-[(3,4-dichlorophényl) acétyl] 8-(1-pyrrolidinyl) quinoléine et de ses sels d'addition avec les acides, caractérisé en ce que l'on utilise au départ une amine de formule

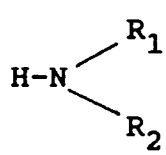

dans laquelle $R_1$ et $R_2$ forment ensemble avec l'atome d'azote auquel ils sont liés, un radical pyrrolidinyle et un composé de formule (IV) ou un dérivé fonctionnel de ce composé, formule (IV) dans laquelle A représente une chaîne $-CH_2-$ et Z représente un radical 3,4-dichloro-phényl.

**7.** Procédé selon la revendication 5, pour la préparation des composés de formule (I) telle que définie à la revendication 1, dont les noms suivent :

- la (4a alpha, 8 alpha, 8a alpha) (±) décahydro 1-[[4- (trifluorométhyl) phényl] acétyl] 8-(1-pyrrolidinyl) quinoléine,
- la (4a alpha, 8 alpha, 8a alpha) (±) décahydro 1-[(4-bromophényl) acétyl) 8-(1-pyrrolidinyl) quinoléine,
- la (4a alpha, 8 alpha, 8a alpha) (±) décahydro 1-[2-(3,4-dichloro-phényl) 1-oxopropyl] 8-(1-pyrrolidinyl) quinoléine,
- la (4a alpha, 8 alpha, 8a alpha) (±) décahydro 1-(3,4-dichlorobenzoyl) 8-(1-pyrrolidinyl) quinoléine,
- la (4a alpha, 8 alpha, 8a alpha) (±) décahydro 1-[(4-benzo [b] thiényl) acétyl] 8-(1-pyrrolidinyl) quinoléine,
- la (4a alpha, 8 alpha, 8a alpha) (±) décahydro 1-[(1-naphtalényl) acétyl] 8-(1-pyrrolidinyl) quinoléine,

ainsi que leurs sels d'addition avec les acides et leurs sels d'ammonium quaternaire, caractérisé en ce que l'on utilise au départ une amine de formule

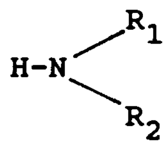

dans laquelle $R_1$ et $R_2$ forment ensemble avec l'atome d'azote auquel ils sont liés, un radical pyrrolidinyle et un composé de formule (IV) ou un dérivé fonctionnel de ce composé, formule (IV) dans laquelle A et Z ont les valeurs correspondant aux composés nommés plus haut.

**8.** A titre de produit industriel nouveau la 8-chloro 5,6,7,8-tétrahydroquinoléine.

**9.** A titre de produits industriels nouveaux, les produits de formules II et III telles que définies à la revendication 1.

**10.** Procédé de préparation de compositions pharmaceutiques, caractérisé en ce que l'on met, à titre de principe actif, l'un au moins des composés de formule (I), telle que définie à la revendication 1, ou l'un au moins de leurs sels pharmaceutiquement acceptables, sous une forme destinée à cet usage.

**11.** Procédé de préparation de compositions pharmaceutiques, caractérisé en ce que l'on met à titre de principe actif, l'un au moins des composés de formule (I), telle que définie à la revendication 2, ou l'un au moins de leurs sels pharmaceutiquement acceptables, sous une forme destinée à cet usage.

**12.** Procédé de préparation de compositions pharmaceutiques, caractérisé en ce que l'on met, à titre de principe actif, l'un au moins des composés de formule (I), telle que définie à la revendication 3, 4 ou 5 ou l'un au moins de leurs sels pharmaceutiquement acceptables, sous une forme destinée à cet usage.

**13.** Procédé selon la revendication 10, caractérisé en ce que le principe actif est choisi dans le groupe constitué par :
- la (4a alpha, 8 alpha, 8a alpha) (±) décahydro 1-[[4-(trifluorométhyl) phényl] acétyl] 8-(1-pyrrolidinyl) quinoléine,
- la (4a alpha, 8 alpha, 8a alpha) (±) décahydro 1-[(4-bromophényl) acétyl] 8-(1-pyrrolidinyl) quinoléine,
- la (4a alpha, 8 alpha, 8a alpha) (±) décahydro 1-[2-(3,4-dichloro-phényl) 1-oxopropyl] 8-(1-pyrrolidinyl) quinoléine,
- la (4a alpha, 8 alpha, 8a alpha) (±) décahydro 1-(3,4-dichlorobenzoyl) 8-(1-pyrrolidinyl) quinoléine,
- la (4a alpha, 8 alpha, 8a alpha) (±) décahydro 1-[(4-benzo [b] thiényl) acétyl] 8-(1-pyrrolidinyl) quinoléine,
- la (4a alpha, 8 alpha, 8a alpha) (±) décahydro 1-[(1-naphtalényl) acétyl] 8-(1-pyrrolidinyl) quinoléine,

ainsi que leurs sels d'addition avec les acides et leurs sels d'ammonium quaternaire.

**14.** Procédé selon la revendication 10, caractérisé en ce que le principe actif est constitué par la (4a alpha, 8 alpha, 8a alpha) (±) décahydro 1-[(3,4-dichlorophényl) acétyl] 8-(1-pyrrolidinyl) quinoléine ou l'un de

ses sels d'addition avec les acides.

## Claims

### Claims for the following Contracting States : BE, CH, DE, FR, GB, IT, LI, LU, NL, SE

1. Compounds of formula (I):

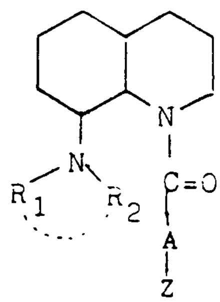

(I)

in which $R_1$ and $R_2$, identical or different, represent a hydrogen atom or an alkyl radical containing 1 to 5 carbon atoms or $R_1$ and $R_2$ form with the nitrogen atom to which they are linked a pyrrolidinyl, piperidinyl or morpholinyl radical, these radicals being optionally substituted by an alkyl or alkoxy radical containing 1 to 5 carbon atoms, A represents a $(CH_2)_n$ chain in which n represents an integer from 0 to 5, or A represents an alkylene chain substituted by an alkyl radical containing in total 2 to 8 carbon atoms, Z represents a phenyl radical optionally substituted by one or more identical or different radicals, chosen from the group constituted by alkyl radicals containing 1 to 5 carbon atoms, alkoxy radicals containing 1 to 5 carbon atoms, halogen atoms, hydroxyl, trifluoromethyl, nitro, amino radicals, monoalkyl- or dialkylamino radicals the alkyl radicals of which contain 1 to 5 carbon atoms, or Z represents a naphthyl radical, or one of the following radicals: indenyl, pyridinyl, thienyl, thiazolyl, oxazolyl, isoxazolyl, imidazolyl, indolyl, quinolyl, benzofuranyl, benzo-[b]-thienyl, benzimidazolyl, benzoxazolyl or benzothiazolyl, all these radicals being optionally substituted by one or more identical or different substituents, chosen from the group constituted by alkyl radicals containing 1 to 5 carbon atoms, alkoxy radicals containing 1 to 5 carbon atoms, trifluoromethyl, nitro, amino radicals, monoalkyl- and dialkylamino radicals the alkyl radicals of which contain 1 to 5 carbon atoms and phenyl radicals optionally substituted by one or more alkyl radicals containing 1 to 5 carbon atoms, alkoxy radicals containing 1 to 5 carbon atoms or halogens, the said compounds of formula (I) being able to be in all the possible enantiomer and diastereoisomer forms and in the form of addition salts with acids or of quaternary ammonium salts.

2. Compounds of formula (I) as defined in claim 1, in which $R_1$ and $R_2$ represent a methyl or ethyl radical, or form with the nitrogen atom to which they are linked a pyrrolidinyl or piperidinyl radical, A represents a $(CH_2)_n$ chain in which n is equal to 0 or 1, or a 1,1-ethanediyl chain, Z represents a phenyl radical, optionally substituted by one or more identical or different radicals, chosen from the group constituted by alkyl radicals containing 1 to 5 carbon atoms, alkoxy radicals containing 1 to 5 carbon atoms, halogen atoms, hydroxyl, trifluoromethyl, nitro, amino radicals, monoalkyl- or dialkylamino radicals the alkyl radicals of which contain 1 to 5 carbon atoms, or Z represents one of the following radicals: naphthyl, indenyl, pyridinyl, thienyl, thiazolyl, oxazolyl, isoxazolyl, imidazolyl, indolyl, quinolyl, benzofuranyl, benzo-[b]-thienyl, benzimidazolyl, benzoxazolyl or benzothiazolyl, all these radicals being optionally substituted by one or more identical or different substituents, chosen from the group constituted by alkyl radicals containing 1 to 5 carbon atoms, alkoxy radicals containing 1 to 5 carbon atoms, trifluoromethyl, nitro, amino radicals, monoalkyl- or dialkylamino radicals the alkyl radicals of which contain 1 to 5 carbon atoms and phenyl radicals optionally substituted by one or more alkyl radicals containing 1 to 5 carbon atoms, alkoxy radicals containing 1 to 5 carbon atoms or halogen atoms, as well as their addition salts with acids and their quaternary ammonium salts.

3. Compounds of formula (I) as defined in claim 2, in which $R_1$, $R_2$ and A have the meaning already indicated and Z represents a phenyl radical, optionally substituted by one or more identical or different substituents, chosen from the group constituted by alkyl radicals containing 1 to 5 carbon atoms, alkoxy radicals containing 1 to 5 carbon atoms, halogen atoms, hydroxyl, trifluoromethyl, nitro, amino radicals,

monoalkyl- or dialkylamino radicals the alkyl radicals of which contain 1 to 5 carbon atoms, or Z represents a naphthyl, pyridinyl, thienyl, indolyl, benzo-[b]-thienyl radical, all these radicals being optionally substituted by one or more identical or different substituents, chosen from the group constituted by alkyl radicals containing 1 to 5 carbon atoms, alkoxy radicals containing 1 to 5 carbon atoms, trifluoromethyl, nitro, amino radicals, monoalkyl- or dialkylamino radicals the alkyl radicals of which contain 1 to 5 carbon atoms and phenyl radicals optionally substituted by one or more alkyl radicals containing 1 to 5 carbon atoms, alkoxy radicals containing 1 to 5 carbon atoms or halogen atoms, as well as their addition salts with acids and their quaternary ammonium salts.

**4.** Compounds of formula (I) as defined in any one of claims 1 to 3, in which $R_1$ and $R_2$ form with the nitrogen atom to which they are linked a pyrrolidinyl radical, A represents a $(CH_2)_n$ chain in which n is equal to 0 or 1, or a 1,1-ethanediyl chain, Z represents a phenyl radical substituted by one or more substituents chosen from the group constituted by halogen atoms and the trifluoromethyl radical or Z represents a naphthyl or benzo-[b]-thienyl radical, as well as their addition salts with acids and their quaternary ammonium salts.

**5.** Any one of the compounds of formula (I) as defined in claim 1, of which the names follow:
- (4a alpha, 8 alpha, 8a alpha) (±) decahydro 1-[(3,4-dichlorophenyl)-acetyl]-8-(1-pyrrolidinyl)-quinoline,
- (4a alpha, 8 alpha, 8a alpha) (±) decahydro 1-[[4-(trifluoromethyl)-phenyl]-acetyl]-8-(1-pyrrolidinyl)-quinoline,
- (4a alpha, 8 alpha, 8a alpha) (±) decahydro 1-[(4-bromophenyl)-acetyl]-8-(1-pyrrolidinyl)-quinoline,
- (4a alpha, 8 alpha, 8a alpha) (±) decahydro 1-[2-(3,4-dichloro-phenyl)-1-oxopropyl]-8-(1-pyrrolidinyl)-quinoline,
- (4a alpha, 8 alpha, 8a alpha) (±) decahydro 1-(3,4-dichlorobenzoyl)-8-(1-pyrrolidinyl) quinoline,
- (4a alpha, 8 alpha, 8a alpha) (±) decahydro 1-[(4-benzo-[b]-thienyl)-acetyl]-8-(1-pyrrolidinyl)-quinoline,
- (4a alpha, 8 alpha, 8a alpha) (±) decahydro 1-[(1-naphthalenyl)-acetyl]-8-(1-pyrrolidinyl)-quinoline,

as well as their addition salts with acids and their quaternary ammonium salts.

**6.** Preparation process for the compounds of formula (I) as defined in claims 1 to 3, characterized in that 8-chloro-5,6,7,8-tetrahydro quinoline of formula:

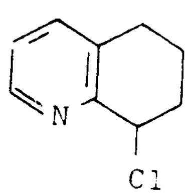

is condensed with an amine of formula:

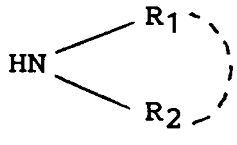

in which $R_1$ and $R_2$ have the meanings indicated previously, in order to obtain a compound of formula (II):

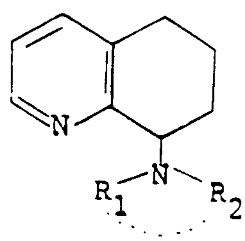

(II)

which is reduced in order to obtain a compound of formula (III):

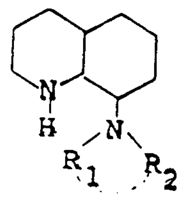

(III)

which is condensed with a compound of formula (IV) or a functional derivative of this compound:

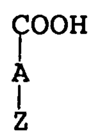

(IV)

in which A and Z have the meanings indicated previously, in order to obtain a compound of formula (I) in all the possible enantiomer and diastereoisomer forms, which is treated, if desired, with a mineral or organic acid in order to obtain a salt or with an alkyl halide in order to obtain a quaternary ammonium salt.

**7.** Preparation process for the compounds of formula (I) as defined in claim 6, characterized in that the reduction of the compound of formula (II) is carried out by a chemical reducing agent and preferably compounds of formula (I) are obtained in which the ring junction is trans and in that the reduction of the compound of formula (II) is carried out by catalytic hydrogenation and preferably compounds of formula (I) are obtained in which the ring junction is cis.

**8.** As medicaments, the products of formula (I) as defined in claims 1 to 4.

**9.** As medicaments, the products claim 5.

**10.** The pharmaceutical compositions containing as active ingredient at least one of the medicaments as defined in claims 8 and 9.

**11.** As a new industrial product, 8-chloro-5,6,7,8-tetrahydro quinoline.

**12.** As new industrial products, the products of formulae (II) and (III) as defined in claim 6.

**Claims for the following Contracting State : AT**

**1.** Preparation process for the compounds of formula (I):

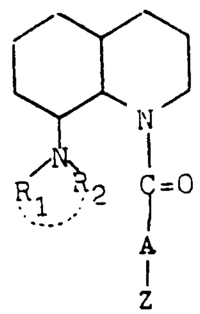

(I)

in which $R_1$ and $R_2$, identical or different, represent a hydrogen atom or an alkyl radical containing 1 to 5 carbon atoms or $R_1$ and $R_2$ form with the nitrogen atom to which they are linked a pyrrolidinyl, piperidinyl or morpholinyl radical, these radicals being optionally substituted by an alkyl or alkoxy radical containing 1 to 5 carbon atoms, A represents a $(CH_2)_n$ chain in which n represents an integer from 0 to 5, or A represents an alkylene chain substituted by an alkyl radical containing in total 2 to 8 carbon atoms, Z represents a phenyl radical optionally substituted by one or more identical or different radicals, chosen from the group constituted by alkyl radicals containing 1 to 5 carbon atoms, alkoxy radicals containing 1 to 5 carbon atoms, halogen atoms, hydroxyl, trifluoromethyl, nitro, amino radicals, monoalkyl- or dialkylamino radicals the alkyl radicals of which contain 1 to 5 carbon atoms, or Z represents a naphthyl radical, or one of the following radicals: indenyl, pyridinyl, thienyl, thiazolyl, oxazolyl, isoxazolyl, imidazolyl, indolyl, quinolyl, benzofuranyl, benzo-[b]-thienyl, benzimidazolyl, benzoxazolyl or benzothiazolyl, all these radicals being optionally substituted by one or more identical or different substituents, chosen from the group constituted by alkyl radicals containing 1 to 5 carbon atoms, alkoxy radicals containing 1 to 5 carbon atoms, trifluoromethyl, nitro, amino radicals, monoalkyl- and dialkylamino radicals the alkyl radicals of which contain 1 to 5 carbon atoms and phenyl radicals optionally substituted by one or more alkyl radicals containing 1 to 5 carbon atoms, alkoxy radicals containing 1 to 5 carbon atoms or halogens, the said compounds of formula (I) being able to be in all the possible enantiomer and diastereoisomer forms and in the form of addition salts with acids or of quaternary ammonium salts, characterized in that 8-chloro-5,6,7,8-tetrahydro quinoline of formula:

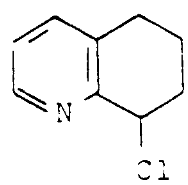

is condensed with an amine of formula:

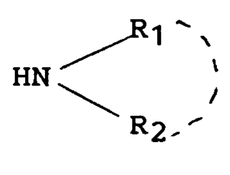

in which $R_1$ and $R_2$ have the meanings indicated previously, in order to obtain a compound of formula (II):

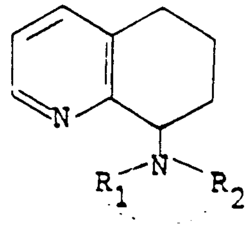

(II)

which is reduced in order to obtain a compound of formula (III):

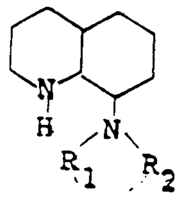

(III)

which is condensed with a compound of formula (IV) or a functional derivative of this compound:

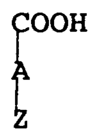

(IV)

in which A and Z have the meanings indicated previously, in order to obtain a compound of formula (I) in all the possible enantiomer and diastereoisomer forms, which is treated, if desired, with a mineral or organic acid in order to obtain a salt or with an alkyl halide in order to obtain a quaternary ammonium salt.

**2.** Preparation process for the compounds of formula (I) as defined in claim 1, characterized in that the reduction of the compound of formula (II) is carried out by a chemical reducing agent and preferably compounds of formula (I) are obtained in which the ring junction is trans and in that the reduction of the compound of formula (II) is carried out by catalytic hydrogenation and preferably compounds of formula (I) are obtained in which the ring junction is cis.

**3.** Process according to claim 1, characterized in that an amine of formula

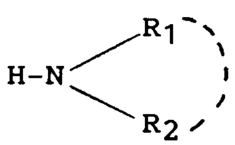

is used at the start, in which $R_1$ and $R_2$ represent a methyl or ethyl radical, or form with the nitrogen atom to which they are linked a pyrrolidinyl or piperidinyl radical, and a compound of formula (IV) or a functional derivative of this compound, formula (IV) in which A represents a $(CH_2)_n$ chain in which n is equal to 0 or 1, or a 1,1-ethanediyl chain, Z represents a phenyl radical optionally substituted by one or more identical or different radicals, chosen from the group constituted by alkyl radicals containing 1 to 5 carbon atoms, alkoxy radicals containing 1 to 5 carbon atoms, halogen atoms, hydroxyl, trifluoromethyl, nitro, amino radicals, monoalkyl- or dialkylamino radicals the alkyl radicals of which contain 1 to 5 carbon atoms, or Z represents a naphthyl radical, or one of the following radicals: indenyl, pyridinyl, thienyl, thiazolyl, oxazolyl, isoxazolyl, imidazolyl, indolyl, quinolyl, benzofuranyl, benzo-[b]-thienyl, benzimidazolyl, benzoxazolyl or benzothiazolyl, all these radicals being optionally substituted by one or more identical or different substituents, chosen from the group constituted by alkyl radicals containing 1 to 5 carbon atoms, alkoxy radicals containing 1 to 5 carbon atoms, trifluoromethyl, nitro, amino radicals, monoalkyl- or dialkylamino radicals the alkyl radicals of which contain 1 to 5 carbon atoms and phenyl radicals optionally substituted by one or more alkyl radicals containing 1 to 5 carbon atoms, alkoxy radicals containing 1 to 5 carbon atoms or halogen atoms.

**4.** Process according to claim 3, characterized in that a compound of formula (IV) or a functional derivative of this compound is used at the start, in which formula (IV) Z represents a phenyl radical optionally substituted by one or more identical or different substituents, chosen from the group constituted by alkyl radicals containing 1 to 5 carbon atoms, alkoxy radicals containing 1 to 5 carbon

atoms, halogen atoms, hydroxyl, trifluoromethyl, nitro, amino radicals, monoalkyl- or dialkylamino radicals the alkyl radicals of which contain 1 to 5 carbon atoms, or Z represents a naphthyl, pyridinyl, thienyl, indolyl or benzo-[b]-thienyl radical, all these radicals being optionally substituted by one or more identical or different substituents, chosen from the group constituted by alkyl radicals containing 1 to 5 carbon atoms, alkoxy radicals containing 1 to 5 carbon atoms, trifluoromethyl, nitro, amino radicals, monoalkyl- or dialkylamino radicals the alkyl radicals of which contain 1 to 5 carbon atoms and phenyl radicals optionally substituted by one or more alkyl radicals containing 1 to 5 carbon atoms, alkoxy radicals containing 1 to 5 carbon atoms or halogen atoms.

**5.** Process according to any one of claims 1, 3 or 4, characterized in that an amine of formula:

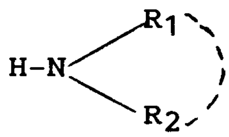

is used at the start, in which $R_1$ and $R_2$ form with the nitrogen atom to which they are linked a pyrrolidinyl radical and a compound of formula (IV) or a functional derivative of this compound, in which formula (IV) A represents a $(CH_2)_n$ chain in which n is equal to 0 or 1, or a 1,1-ethanediyl chain, Z represents a phenyl radical substituted by one or more substituents chosen from the group constituted by halogen atoms and the trifluoromethyl radical or Z represents a naphthyl or benzo-[b]-thienyl radical.

**6.** Process according to claim 5, for the preparation of (4a alpha, 8 alpha, 8a alpha) (±) decahydro 1-[(3,4-dichlorophenyl)-acetyl]-8-(1-pyrrolidinyl)-quinoline and its addition salts with acids, characterized in that an amine of formula:

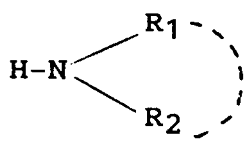

is used at the start, in which $R_1$ and $R_2$ form with the nitrogen atom to which they are linked a pyrrolidinyl radical and a compound of formula (IV) or a functional derivative of this compound, in which formula (IV) A represents a $-CH_2$-chain and Z represents a 3,4-dichloro-phenyl radical.

**7.** Process according to claim 5, for the preparation of compounds of formula (I) as defined in claim 1, the names of which follow:

- (4a alpha, 8 alpha, 8a alpha) (±) decahydro 1-[[4-(trifluoromethyl)-phenyl]-acetyl]-8-(1-pyrrolidinyl)-quinoline,
- (4a alpha, 8 alpha, 8a alpha) (±) decahydro 1-[(4-bromophenyl)-acetyl]-8-(1-pyrrolidinyl)-quinoline,
- (4a alpha, 8 alpha, 8a alpha) (±) decahydro 1-[2-(3,4-dichloro-phenyl)-1-oxopropyl]-8-(1-pyrrolidinyl)-quinoline,
- (4a alpha, 8 alpha, 8a alpha) (±) decahydro 1-(3,4-dichlorobenzoyl)-8-(1-pyrrolidinyl) quinoline,
- (4a alpha, 8 alpha, 8a alpha) (±) decahydro 1-[(4-benzo-[b]-thienyl)-acetyl]-8-(1-pyrrolidinyl)-quinoline,
- (4a alpha, 8 alpha, 8a alpha) (±) decahydro 1-[(1-naphthalenyl)-acetyl]-8-(1-pyrrolidinyl)-quinoline,

as well as their addition salts with acids and their quaternary ammonium salts, characterized in that an amine of formula:

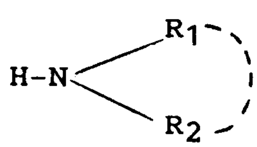

is used at the start, in which $R_1$ and $R_2$ form with the nitrogen atom to which they are linked a

pyrrolidinyl radical and a compound of formula (IV) or a functional derivative of this compound, in which formula (IV) A and Z have the values corresponding to the compounds named above.

**Claims for the following Contracting State : ES**

**1.** Preparation process for compounds of formula (I):

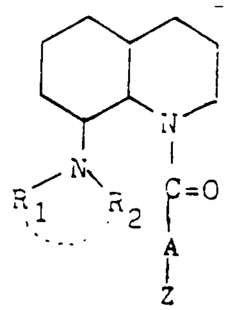

(I)

in which $R_1$ and $R_2$, identical or different, represent a hydrogen atom or an alkyl radical containing 1 to 5 carbon atoms or $R_1$ and $R_2$ form with the nitrogen atom to which they are linked a pyrrolidinyl, piperidinyl or morpholinyl radical, these radicals being optionally substituted by an alkyl or alkoxy radical containing 1 to 5 carbon atoms, A represents a $(CH_2)_n$ chain in which n represents an integer from 0 to 5, or A represents an alkylene chain substituted by an alkyl radical containing in total 2 to 8 carbon atoms, Z represents a phenyl radical optionally substituted by one or more identical or different radicals, chosen from the group constituted by alkyl radicals containing 1 to 5 carbon atoms, alkoxy radicals containing 1 to 5 carbon atoms, halogen atoms, hydroxyl, trifluoromethyl, nitro, amino radicals, monoalkyl- or dialkylamino radicals the alkyl radicals of which contain 1 to 5 carbon atoms, or Z represents a naphthyl radical, or one of the following radicals: indenyl, pyridinyl, thienyl, thiazolyl, oxazolyl, isoxazolyl, imidazolyl, indolyl, quinolyl, benzofuranyl, benzo-[b]-thienyl, benzimidazolyl, benzoxazolyl or benzothiazolyl, all these radicals being optionally substituted by one or more identical or different substituents, chosen from the group constituted by alkyl radicals containing 1 to 5 carbon atoms, alkoxy radicals containing 1 to 5 carbon atoms, trifluoromethyl, nitro, amino radicals, monoalkyl- and dialkylamino radicals the alkyl radicals of which contain 1 to 5 carbon atoms and phenyl radicals optionally substituted by one or more alkyl radicals containing 1 to 5 carbon atoms, alkoxy radicals containing 1 to 5 carbon atoms or halogens, the said compounds of formula (I) being able to be in all the possible enantiomer and diastereoisomer forms and in the form of addition salts with acids or of quaternary ammonium salts, characterized in that 8-chloro-5,6,7,8-tetrahydro quinoline of formula:

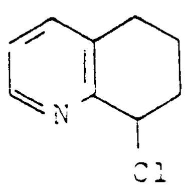

is condensed with an amine of formula:

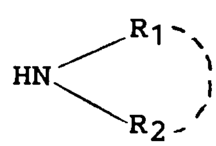

in which $R_1$ and $R_2$ have the meanings indicated previously, in order to obtain a compound of formula (II):

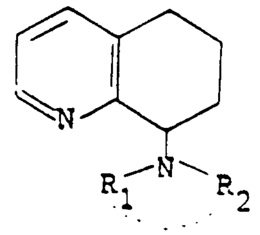

(II)

which is reduced in order to obtain a compound of formula (III):

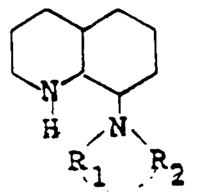

(III)

which is condensed with a compound of formula (IV) or a functional derivative of this compound:

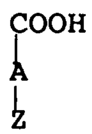

(IV)

in which A and Z have the meanings indicated previously, in order to obtain a compound of formula (I) in all the possible enantiomer and diastereoisomer forms, which is treated, if desired, with a mineral or organic acid in order to obtain a salt or with an alkyl halide in order to obtain a quaternary ammonium salt.

**2.** Preparation process for the compounds of formula (I) as defined in claim 1, characterized in that the reduction of the compound of formula (II) is carried out by a chemical reducing agent and preferably compounds of formula (I) are obtained in which the ring junction is trans and in that the reduction of the compound of formula (II) is carried out by catalytic hydrogenation and preferably compounds of formula (I) are obtained in which the ring junction is cis.

**3.** Process according to claim 1, characterized in that an amine of formula

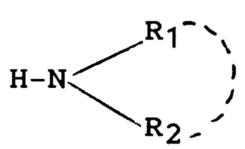

is used at the start, in which $R_1$ and $R_2$ represent a methyl or ethyl radical, or form with the nitrogen atom to which they are linked a pyrrolidinyl or piperidinyl radical, and a compound of formula (IV) or a functional derivative of this compound, formula (IV) in which A represents a $(CH_2)_n$ chain in which n is equal to 0 or 1, or a 1,1-ethanediyl chain, Z represents a phenyl radical optionally substituted by one or more identical or different radicals, chosen from the group constituted by alkyl radicals containing 1 to 5 carbon atoms, alkoxy radicals containing 1 to 5 carbon atoms, halogen atoms, hydroxyl, trifluoromethyl, nitro, amino radicals, monoalkyl- or dialkylamino radicals the alkyl radicals of which contain 1 to 5 carbon atoms, or Z represents a naphthyl radical, or one of the following radicals: indenyl, pyridinyl, thienyl, thiazolyl, oxazolyl, isoxazolyl, imidazolyl, indolyl, quinolyl, benzofuranyl, benzo-[b]-thienyl, benzimidazolyl, benzoxazolyl or benzothiazolyl, all these radicals being optionally substituted by one or more identical or different substituents, chosen from the group constituted by alkyl radicals containing 1 to 5 carbon atoms, alkoxy radicals containing 1 to 5 carbon atoms,

trifluoromethyl, nitro, amino radicals, monoalkyl- or dialkylamino radicals the alkyl radicals of which contain 1 to 5 carbon atoms and phenyl radicals optionally substituted by one or more alkyl radicals containing 1 to 5 carbon atoms, alkoxy radicals containing 1 to 5 carbon atoms or halogen atoms.

**4.** Process according to claim 3, characterized in that a compound of formula (IV) or a functional derivative of this compound is used at the start, in which formula (IV) Z represents a phenyl radical optionally substituted by one or more identical or different substituents, chosen from the group constituted by alkyl radicals containing 1 to 5 carbon atoms, alkoxy radicals containing 1 to 5 carbon atoms, halogen atoms, hydroxyl, trifluoromethyl, nitro, amino radicals, monoalkyl- or dialkylamino radicals the alkyl radicals of which contain 1 to 5 carbon atoms, or Z represents a naphthyl, pyridinyl, thienyl, indolyl or benzo-[b]-thienyl radical, all these radicals being optionally substituted by one or more identical or different substituents, chosen from the group constituted by alkyl radicals containing 1 to 5 carbon atoms, alkoxy radicals containing 1 to 5 carbon atoms, trifluoromethyl, nitro, amino radicals, monoalkyl- or dialkylamino radicals the alkyl radicals of which contain 1 to 5 carbon atoms and phenyl radicals optionally substituted by one or more alkyl radicals containing 1 to 5 carbon atoms, alkoxy radicals containing 1 to 5 carbon atoms or halogen atoms.

**5.** Process according to any one of claims 1, 3 or 4, characterized in that an amine of formula:

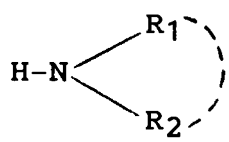

is used at the start, in which $R_1$ and $R_2$ form with the nitrogen atom to which they are linked a pyrrolidinyl radical and a compound of formula (IV) or a functional derivative of this compound, in which formula (IV) A represents a $(CH_2)_n$ in which n is equal to 0 or 1, or a 1,1-ethanediyl chain, Z represents a phenyl radical substituted by one or more substituents chosen from the group constituted by halogen atoms and the trifluoromethyl radical or Z represents a naphthyl or benzo-[b]-thienyl radical.

**6.** Process according to claim 5, for the preparation of (4a alpha, 8 alpha, 8a alpha) (±) decahydro 1-[(3,4-dichlorophenyl)-acetyl]-8-(1-pyrrolidinyl)-quinoline and its addition salts with acids, characterized in that an amine of formula:

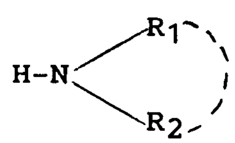

in which $R_1$ and $R_2$ form with the nitrogen atom to which they are linked a pyrrolidinyl radical and a compound of formula (IV) or a functional derivative of this compound, in which formula (IV) A represents a $-CH_2-$ chain and Z represents a 3,4-dichloro-phenyl radical.

**7.** Process according to claim 5, for the preparation of compounds of formula (I) as defined in claim 1, the names of which follow:

- (4a alpha, 8 alpha, 8a alpha) (±) decahydro 1-[[4-(trifluoromethyl)-phenyl]-acetyl]-8-(1-pyrrolidinyl)-quinoline,
- (4a alpha, 8 alpha, 8a alpha) (±) decahydro 1-[(4-bromophenyl)-acetyl]-8-(1-pyrrolidinyl)-quinoline,
- (4a alpha, 8 alpha, 8a alpha) (±) decahydro 1-[2-(3,4-dichloro-phenyl)-1-oxopropyl]-8-(1-pyrrolidinyl)-quinoline,
- (4a alpha, 8 alpha, 8a alpha) (±) decahydro 1-(3,4-dichlorobenzoyl)-8-(1-pyrrolidinyl) quinoline,
- (4a alpha, 8 alpha, 8a alpha) (±) decahydro 1-[(4-benzo-[b]-thienyl)-acetyl]-8-(1-pyrrolidinyl)-quinoline,
- (4a alpha, 8 alpha, 8a alpha) (±) decahydro 1-[(1-naphthalenyl)-acetyl]-8-(1-pyrrolidinyl)-quinoline,

as well as their addition salts with acids and their quaternary ammonium salts, characterized in that an amine of formula:

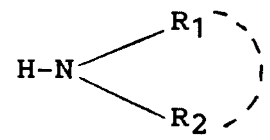

is used at the start, in which $R_1$ and $R_2$ form with the nitrogen atom to which they are linked a pyrrolidinyl radical and a compound of formula (IV) or a functional derivative of this compound, in which formula (IV) A and Z have the values corresponding to the compounds named above.

**Claims for the following Contracting State : GR**

**1.** Preparation process for compounds of formula (I):

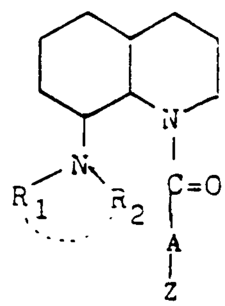

(I)

in which $R_1$ and $R_2$, identical or different, represent a hydrogen atom or an alkyl radical containing 1 to 5 carbon atoms or $R_1$ and $R_2$ form with the nitrogen atom to which they are linked a pyrrolidinyl, piperidinyl or morpholinyl radical, these radicals being optionally substituted by an alkyl or alkoxy radical containing 1 to 5 carbon atoms, A represents a $(CH_2)_n$ chain in which n represents an integer from 0 to 5, or A represents an alkylene chain substituted by an alkyl radical containing in total 2 to 8 carbon atoms, Z represents a phenyl radical optionally substituted by one or more identical or different radicals, chosen from the group constituted by alkyl radicals containing 1 to 5 carbon atoms, alkoxy radical containing 1 to 5 carbon atoms, halogen atoms, hydroxyl, trifluoromethyl, nitro, amino radicals, monoalkyl- or dialkylamino radicals the alkyl radicals of which contain 1 to 5 carbon atoms, or Z represents a naphthyl radical, or one of the following radicals: indenyl, pyridinyl, thienyl, thiazolyl, oxazolyl, isoxazolyl, imidazolyl, indolyl, quinolyl, benzofuranyl, benzo-[b]-thienyl, benzimidazolyl, benzoxazolyl or benzothiazolyl, all these radicals being optionally substituted by one or more identical or different substituents, chosen from the group constituted by alkyl radicals containing 1 to 5 carbon atoms, alkoxy radicals containing 1 to 5 carbon atoms, trifluoromethyl, nitro, amino radicals, monoalkyl- and dialkylamino radicals the alkyl radicals of which contain 1 to 5 carbon atoms and phenyl radicals optionally substituted by one or more alkyl radicals containing 1 to 5 carbon atoms, alkoxy radicals containing 1 to 5 carbon atoms or halogens, the said compounds of formula (I) being able to be in all the possible enantiomer and diastereoisomer forms and in the form of addition salts with acids or of quaternary ammonium salts, characterized in that 8-chloro-5,6,7,8-tetrahydro quinoline of formula:

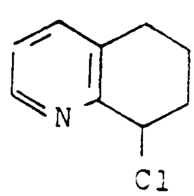

is condensed with an amine of formula:

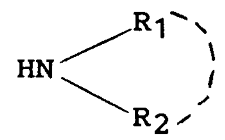

in which $R_1$ and $R_2$ have the meanings indicated previously, in order to obtain a compound of formula (II):

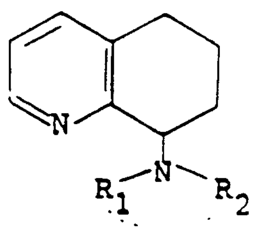

(II)

which is reduced in order to obtain a compound of formula (III):

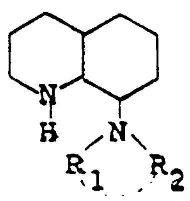

(III)

which is condensed with a compound of formula (IV) or a functional derivative of this compound:

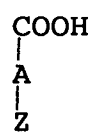

(IV)

in which A and Z have the meanings indicated previously, in order to obtain a compound of formula (I) in all the possible enantiomer and diastereoisomer forms, which is treated, if desired, with a mineral or organic acid in order to obtain a salt or with an alkyl halide in order to obtain a quaternary ammonium salt.

**2.** Preparation process for the compounds of formula (I) as defined in claim 1, characterized in that the reduction of the compound of formula (II) is carried out by a chemical reducing agent and preferably compounds of formula (I) are obtained in which the ring junction is trans and in that the reduction of the compound of formula (II) is carried out by catalytic hydrogenation and preferably compounds of formula (I) are obtained in which the ring junction is cis.

**3.** Process according to claim 1, characterized in that an amine of formula

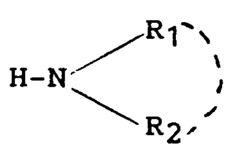

is used at the start, in which $R_1$ and $R_2$ represent a methyl or ethyl radical, or form with the nitrogen atom to which they are linked a pyrrolidinyl or piperidinyl radical, and a compound of formula (IV) or a

functional derivative of this compound, formula (IV) in which A represents a $(CH_2)_n$ chain in which n is equal to 0 or 1, or a 1,1-ethanediyl chain, Z represents a phenyl radical optionally substituted by one or more identical or different radicals, chosen from the group constituted by alkyl radicals containing 1 to 5 carbon atoms, alkoxy radicals containing 1 to 5 carbon atoms, halogen atoms, hydroxyl, trifluoromethyl, nitro, amino radicals, monoalkyl- or dialkylamino radicals the alkyl radicals of which contain 1 to 5 carbon atoms, or Z represents a naphthyl radical, or one of the following radicals: indenyl, pyridinyl, thienyl, thiazolyl, oxazolyl, isoxazolyl, imidazolyl, indolyl, quinolyl, benzofuranyl, benzo-[b]-thienyl, benzimidazolyl, benzoxazolyl or benzothiazolyl, all these radicals being optionally substituted by one or more identical or different substituents, chosen from the group constituted by alkyl radicals containing 1 to 5 carbon atoms, alkoxy radicals containing 1 to 5 carbon atoms, trifluoromethyl, nitro, amino radicals, monoalkyl- or dialkylamino radicals the alkyl radicals of which contain 1 to 5 carbon atoms and phenyl radicals optionally substituted by one or more alkyl radicals containing 1 to 5 carbon atoms, alkoxy radicals containing 1 to 5 carbon atoms or halogen atoms.

4. Process according to claim 3, characterized in that a compound of formula (IV) or a functional derivative of this compound is used at the start, in which formula (IV) Z represents a phenyl radical optionally substituted by one or more identical or different substituents, chosen from the group constituted by alkyl radicals containing 1 to 5 carbon atoms, alkoxy radicals containing 1 to 5 carbon atoms, halogen atoms, hydroxyl, trifluoromethyl, nitro, amino radicals, monoalkyl- or dialkylamino radicals the alkyl radicals of which contain 1 to 5 carbon atoms, or Z represents a naphthyl, pyridinyl, thienyl, indolyl or benzo-[b]-thienyl radical, all these radicals being optionally substituted by one or more identical or different substituents, chosen from the group constituted by alkyl radicals containing 1 to 5 carbon atoms, alkoxy radicals containing 1 to 5 carbon atoms, trifluoromethyl, nitro, amino radicals, monoalkyl- or dialkylamino radicals the alkyl radicals of which contain 1 to 5 carbon atoms and phenyl radicals optionally substituted by one or more alkyl radicals containing 1 to 5 carbon atoms, alkoxy radicals containing 1 to 5 carbon atoms or halogen atoms.

5. Process according to any one of claims 1, 3 or 4, characterized in that an amine of formula:

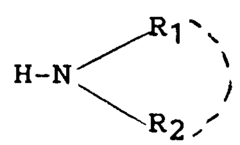

is used at the start, in which $R_1$ and $R_2$ form with the nitrogen atom to which they are linked a pyrrolidinyl radical and a compound of formula (IV) or a functional derivative of this compound, in which formula (IV) A represents a $(CH_2)_n$ chain in which n is equal to 0 or 1, or a 1,1-ethanediyl chain, Z represents a phenyl radical substituted by one or more substituents chosen from the group constituted by halogen atoms and the trifluoromethyl radical or Z represents a naphthyl or benzo-[b]-thienyl radical.

6. Process according to claim 5, for the preparation of (4a alpha, 8 alpha, 8a alpha) (±) decahydro 1-[(3,4-dichlorophenyl)-acetyl]-8-(1-pyrrolidinyl)-quinoline and its addition salts with acids, characterized in that an amine of formula:

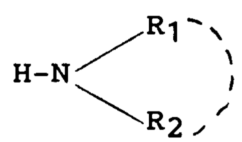

is used at the start, in which $R_1$ and $R_2$ form with the nitrogen atom to which they are linked a pyrrolidinyl radical and a compound of formula (IV) or a functional derivative of this compound, in which formula (IV) A represents a $-CH_2$-chain and Z represents a 3,4-dichloro-phenyl radical.

7. Process according to claim 5, for the preparation of compounds of formula (I) as defined in claim 1, of which the names follow:

- (4a alpha, 8 alpha, 8a alpha) (±) decahydro 1-[[4-(trifluoromethyl)-phenyl]-acetyl]-8-(1-pyrrolidinyl)-quinoline,
- (4a alpha, 8 alpha, 8a alpha) (±) decahydro 1-[(4-bromophenyl)-acetyl]-8-(1-pyrrolidinyl)-quinoline,
- (4a alpha, 8 alpha, 8a alpha) (±) decahydro 1-[2-(3,4-dichloro-phenyl)-1-oxopropyl]-8-(1-pyrrolidinyl)-quinoline,
- (4a alpha, 8 alpha, 8a alpha) (±) decahydro 1-(3,4-dichlorobenzoyl)-8-(1-pyrrolidinyl) quinoline,
- (4a alpha, 8 alpha, 8a alpha) (±) decahydro 1-[(4-benzo-[b]-thienyl)-acetyl]-8-(1-pyrrolidinyl)-quinoline,
- (4a alpha, 8 alpha, 8a alpha) (±) decahydro 1-[(1-naphthalenyl)-acetyl]-8-(1-pyrrolidinyl)-quinoline,

as well as their addition salts with acids and their quaternary ammonium salts, characterized in that an amine of formula:

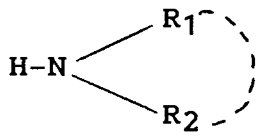

is used at the start, in which $R_1$ and $R_2$ form with the nitrogen atom to which they are linked a pyrrolidinyl radical and a compound of formula (IV) or a functional derivative of this compound, in which formula (IV) A and Z have the values corresponding to the compounds named above.

**8.** As a new industrial product, 8-chloro-5,6,7,8-tetrahydro quinoline.

**9.** As new industrial products, the products of formulae (II) and (III) as defined in claim 1.

**10.** Preparation process for pharmaceutical compositions, characterized in that at least one of the compounds of formula (I) as defined in claim 1, or at least one of their pharmaceutically acceptable salts, is used as active ingredient in a form intended for this use.

**11.** Preparation process for pharmaceutical compositions, characterized in that at least one of the compounds of formula (I) as defined 2, or at least one of their pharmaceutically acceptable salts, is used as active ingredient in a form intended for this use.

**12.** Preparation process for pharmaceutical compositions, characterized in that at least one of the compounds of formula (I) as defined in claim 3, 4 or 5 or at least one of their pharmaceutically acceptable salts, is used as active ingredient in a form intended for this use.

**13.** Process according to claim 10, characterized in that the active ingredient is chosen from the group constituted by:
- (4a alpha, 8 alpha, 8a alpha) (±) decahydro 1-[[4-(trifluoromethyl)-phenyl]-acetyl]-8-(1-pyrrolidinyl)-quinoline,
- (4a alpha, 8 alpha, 8a alpha) (±) decahydro 1-[(4-bromophenyl)-acetyl]-8-(1-pyrrolidinyl)-quinoline,
- (4a alpha, 8 alpha, 8a alpha) (±) decahydro 1-[2-(3,4-dichloro-phenyl)-1-oxopropyl]-8-(1-pyrrolidinyl)-quinoline,
- (4a alpha, 8 alpha, 8a alpha) (±) decahydro 1-(3,4-dichlorobenzoyl)-8-(1-pyrrolidinyl) quinoline,
- (4a alpha, 8 alpha, 8a alpha) (±) decahydro 1-[(4-benzo-[b]-thienyl)-acetyl]-8-(1-pyrrolidinyl)-quinoline,
- (4a alpha, 8 alpha, 8a alpha) (±) decahydro 1-[(1-naphthalenyl)-acetyl]-8-(1-pyrrolidinyl)-quinoline,

as well as their addition salts with acids and their quaternary ammonium salts.

**14.** Process according to claim 10, characterized in that the active ingredient is constituted by (4a alpha, 8 alpha, 8a alpha) (±) decahydro 1-[(3,4-dichlorophenyl)-acetyl]-8-(1-pyrrolidinyl)-quinoline or one of its addition salts with acids.

**Patentansprüche**
**Patentansprüche für folgende Vertragsstaaten : BE, CH, DE, FR, GB, IT, LI, LU, NL, SE**

**1.** Verbindungen der Formel (I)

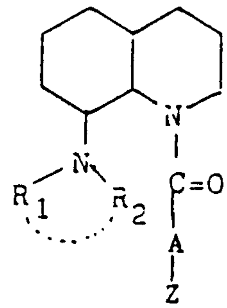

(I)

worin $R_1$ und $R_2$, die gleich oder verschieden sind, ein Wasserstoffatom oder einen Alkylrest mit 1 bis 5 Kohlenstoffatomen bedeuten oder $R_1$ und $R_2$ gemeinsam mit dem Stickstoffatom, an das sie gebunden sind, einen Pyrrolidinyl-, Piperazinyl-, Piperidinyl- oder Morpholinylrest bilden, wobei diese Reste gegebenenfalls durch einen Alkyl-oder Alkoxyrest mit 1 bis 5 Kohlenstoffatomen substituiert sind; A eine Kette $(CH_2)_n$ wiedergibt, worin n eine Zahl von 0 bis 5 bedeutet, oder A eine durch einen Alkylrest mit insgesamt 2 bis 8 Kohlenstoffatomen substituierte Alkylenkette bedeutet; Z für einen Phenylrest steht, der gegebenenfalls durch einen oder mehrere gleiche oder verschiedene Reste substituiert ist, ausgewählt unter den Alkylresten mit 1 bis 5 Kohlenstoffatomen, den Alkoxyresten mit 1 bis 5 Kohlenstoffatomen, den Halogenatomen, den Hydroxyl-, Trifluormethyl-, Nitro-, Amino-, Monoalkyl- oder Dialkylaminoresten, deren Alkylgruppen 1 bis 5 Kohlenstoffatome enthalten, oder Z für einen Naphthyl-, einen Indenyl-, Pyridinyl-, Thienyl-, Thiazolyl-, Oxazolyl-, Isoxazolyl-, Imidazolyl-, Indolyl-, Chinolyl-, Benzofuranyl-, Benzo[b]thienyl-, Benzimidazolyl-, Benzoxazolyl- oder Benzothiazolylrest steht, wobei alle diese Reste gegebenenfalls durch einen oder mehrere gleiche oder verschiedene Substituenten substituiert sind, ausgewählt unter den Alkylresten mit 1 bis 5 Kohlenstoffatomen, den Alkoxyresten mit 1 bis 5 Kohlenstoffatomen, den Trifluormethyl-, Nitro-, Amino-, Monoalkyl- oder Dialkylaminoresten, deren Alkylgruppen 1 bis 5 Kohlenstoffatome aufweisen, und dem Phenylrest, der gegebenenfalls durch einen oder mehrere Alkylreste mit 1 bis 5 Kohlenstoffatomen, Alkoxyreste mit 1 bis 5 Kohlenstoffatomen oder Halogenen substituiert ist, wobei die Verbindungen der Formel (I) in sämtlichen möglichen enantiomeren und diastereomeren Formen und in Form der Additionssalze mit Säuren oder der quaternären Ammoniumsalze vorliegen können.

**2.** Verbindungen der Formel (I) gemäß Anspruch 1, worin $R_1$ und $R_2$ einen Methyl- oder Ethylrest bedeuten oder gemeinsam mit dem Stickstoffatom, an das sie gebunden sind, einen Pyrrolidinyl- oder Piperidinylrest bilden, A eine Kette $(CH_2)_n$, worin n für 0 oder 1 steht, oder eine 1,1-Ethandiylkette wiedergibt, Z einen Phenylrest bedeutet, der gegebenenfalls durch einen oder mehrere gleiche oder verschiedene Reste substituiert ist, ausgewählt unter den Alkylresten mit 1 bis 5 Kohlenstoffatomen, den Alkoxyresten mit 1 bis 5 Kohlenstoffatomen, den Halogenatomen, den Hydroxyl-, Trifluormethyl-, Nitro-, Amino-, Monoalkyl- oder Dialkylaminoresten, deren Alkylgruppen 1 bis 5 Kohlenstoffatome aufweisen, oder Z einen Naphthylrest, einen Indenyl-, Pyridinyl-, Thienyl-, Thiazolyl-, Oxazolyl-, Isoxazolyl-, Imidazolyl-, Indolyl-, Chinolyl-, Benzofuranyl-, Benzo[b]thienyl-, Benzimidazolyl-, Benzoxazolyl- oder Benzothiazolylrest bedeutet, wobei alle diese Reste gegebenenfalls durch einen oder mehrere gleiche oder verschiedene Substituenten substituiert sind, ausgewählt unter den Alkylresten mit 1 bis 5 Kohlenstoffatomen, den Alkoxyresten mit 1 bis 5 Kohlenstoffatomen, den Trifluormethyl-, Nitro-, Amino-, Monoalkyl- oder Dialkylaminoresten, deren Alkylgruppen 1 bis 5 Kohlenstoffatome aufweisen, und dem Phenylrest, der gegebenenfalls durch einen oder mehrere Alkylreste mit 1 bis 5 Kohlenstoffatomen, Alkoxyreste mit 1 bis 5 Kohlenstoffatomen oder Halogene substituiert ist, sowie deren Additionssalze mit Säuren und deren quaternäre Ammoniumsalze.

**3.** Verbindungen der Formel (I), wie in Anspruch 2 definiert, worin $R_1$, $R_2$ und A die angegebene Bedeutung besitzen und Z einen Phenylrest bedeutet, der gegebenenfalls durch einen oder mehrere gleiche oder verschiedene Substituenten substituiert ist, ausgewählt unter den Alkylresten mit 1 bis 5 Kohlenstoffatomen, den Alkoxyresten mit 1 bis 5 Kohlenstoffatomen, den Halogenatomen, den Hydroxyl-, Trifluormethyl-, Nitro-, Amino-, Monoalkyl- oder Dialkylaminoresten, deren Alkylgruppen 1 bis 5 Kohlenstoffatome aufweisen, oder Z einen Naphthyl-, Pyridinyl-, Thienyl-, Indolyl-, Benzo[b]-thienyl-Rest bedeutet, wobei alle diese Reste gegebenenfalls durch einen oder mehrere gleiche oder verschiedene Substituenten substituiert sind,ausgewählt unter den Alkylresten mit 1 bis 5 Kohlenstoffatomen, den Alkoxyresten mit 1 bis 5 Kohlenstoffatomen, den Trifluormethyl-, Nitro-, Amino-, Monoalkyl- oder Dialkylaminoresten, deren Alkylgruppen 1 bis 5 Kohlenstoffatome aufweisen, und dem Phenylrest, der gegebenenfalls durch einen oder mehrere Alkylreste mit 1 bis 5 Kohlenstoffatomen, Alkoxyreste mit 1 bis 5 Kohlenstoffatomen oder Halogene substituiert ist, sowie deren Additionssalze mit Säuren und deren quaternäre Ammoniumsalze.

4. Verbindungen der Formel (I), wie in einem der Ansprüche 1 bis 3 definiert, worin $R_1$ und $R_2$ gemeinsam mit dem Stickstoffatom, an das sie gebunden sind,einen Pyrrolidinylrest bilden, A für eine Kette $(CH_2)_n$,worin n 0 oder 1 bedeutet, oder eine 1,1-Ethandiylkette steht, Z einen Phenylrest wiedergibt, der durch einen oder mehrere Substituenten, ausgewählt unter den Halogenatomen und der Trifluormethylgruppe, substituiert ist, oder Z einen Naphthyl- oder Benzo[b]thienylrest bedeutet, sowie deren Additionssalze mit Säuren und deren quaternäre Ammoniumsalze.

5. Verbindungen der Formel (I), wie in Anspruch 1 definiert, mit den folgenden Bezeichnungen:
(4aα,8α,8aα)-(±)-Decahydro-1-[(3,4-dichlorphenyl)-acetyl]-8-(1-pyrrolidinyl)-chinolin,
(4aα,8α,8aα)-(±)-Decahydro-1-[[4-(trifluormethyl)-phenyl]-acetyl]-8-(1-pyrrolidinyl)-chinolin,
(4aα,8α,8aα)-(±)-Decahydro-1-[(4-bromphenyl)-acetyl]-8-(1-pyrrolidinyl)-chinolin,
(4aα,8α,8aα)-(±)-Decahydro-1-[2-(3,4-dichlorphenyl)-1-oxopropyl]-8-(1-pyrrolidinyl)-chinolin,
(4aα,8α,8aα)-(±)-Decahydro-1-(3,4-dichlorbenzoyl)-8-(1-pyrrolidinyl)-chinolin,
(4aα,8α,8aα)-(±)-Decahydro-1-[(4-benzo[b]thienyl)-acetyl]-8-(1-pyrrolidinyl)-chinolin,
(4aα,8α,8aα)-(±)-Decahydro-1-[(1-naphthalinyl)-acetyl]-8-(1-pyrrolidinyl)-chinolin,
sowie deren Additionssalze mit Säuren und deren quaternäre Ammoniumsalze.

6. Verfahren zur Herstellung von Verbindungen der Formel (I), wie in den Ansprüchen 1 bis 3 definiert, dadurch **gekennzeichnet,** daß man 8-Chlor-5,6,7,8-tetrahydrochinolin der Formel

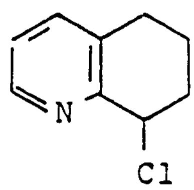

mit einem Amin der Formel

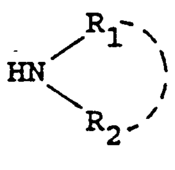

worin $R_1$ und $R_2$ die vorstehend angegebenen Bedeutungen besitzen, kondensiert, um zu einer Verbindung der Formel II

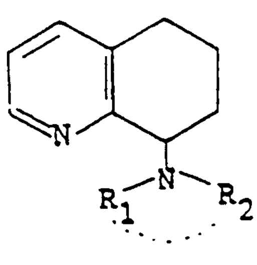

(II)

zu gelangen, welche man reduziert, um eine Verbindung der Formel III

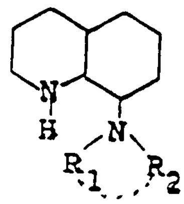

(III)

zu erhalten, die man mit einer Verbindung der Formel IV oder einem funktionellen Derivat dieser Verbindung

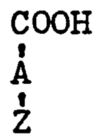

(IV)

worin A und Z die vorstehend angegebenen Bedeutungen haben, kondensiert, um zu einer Verbindung der Formel (I) in sämtlichen ihrer möglichen enantiomeren und diastereomeren Formen zu gelangen, die man gewünschtenfalls mit einer Mineral- oder organischen Säure behandelt, um ein Salz zu erhalten, oder mit einem Alkylhalogenid, um ein quaternäres Ammoniumsalz zu erhalten.

7. Verfahren zur Herstellung der Verbindungen der Formel (I), wie in Anspruch 6 definiert, dadurch gekennzeichnet, daß man die Reduktion der Verbindung der Formel II mit einem chemischen Reduktionsmittel durchführt und man bevorzugt die Verbindungen der Formel (I) erhält, worin die Anknüpfung des Rings trans-ständig ist, und daß man die Reduktion der Verbindung der Formel II durch katalytische Hydrierung bewirkt und vorzugsweise Verbindungen der Formel (I) erhält, worin die Anknüpfung des Rings cis-ständig ist.

8. Als Arzneimittel die Produkte der Formel (I), wie in den Ansprüchen 1 bis 4 definiert.

9. Als Arzneimittel die Produkte gemäß Anspruch 5.

10. Pharmazeutische Zusammensetzungen, enthaltend als Wirkstoff zumindest eines der Arzneimittel, wie in den Ansprüchen 8 und 9 definiert.

11. Als neues, industrielles Produkt das 8-Chlor-5,6,7,8-tetrahydrochinolin.

12. Als neue, industrielle Produkte die Produkte der Formeln II und III, wie in Anspruch 6 definiert.

**Patentansprüche für folgenden Vertragsstaat : AT**

1. Verfahren zur Herstellung der Verbindungen der Formel (I)

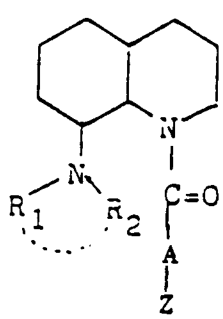

(I)

worin $R_1$ und $R_2$, die gleich oder verschieden sind, ein Wasserstoffatom oder einen Alkylrest mit 1 bis

5 Kohlenstoffatomen bedeuten oder $R_1$ und $R_2$ gemeinsam mit dem Stickstoffatom, an das sie gebunden sind, einen Pyrrolidinyl-, Piperazinyl-, Piperidinyl- oder Morpholinylrest bilden, wobei diese Reste gegebenenfalls durch einen Alkyl-oder Alkoxyrest mit 1 bis 5 Kohlenstoffatomen substituiert sind; A eine Kette $(CH_2)_n$ wiedergibt, worin n eine Zahl von 0 bis 5 bedeutet, oder A eine durch einen Alkylrest mit insgesamt 2 bis 8 Kohlenstoffatomen substituierte Alkylenkette bedeutet; Z für einen Phenylrest steht, der gegebenenfalls durch einen oder mehrere gleiche oder verschiedene Reste substituiert ist, ausgewählt unter den Alkylresten mit 1 bis 5 Kohlenstoffatomen, den Alkoxyresten mit 1 bis 5 Kohlenstoffatomen, den Halogenatomen, den Hydroxyl-, Trifluormethyl-, Nitro-, Amino-, Monoalkyl- oder Dialkylaminoresten, deren Alkylgruppen 1 bis 5 Kohlenstoffatome enthalten, oder Z für einen Naphthyl-, einen Indenyl-, Pyridinyl-, Thienyl-, Thiazolyl-, Oxazolyl-, Isoxazolyl-, Imidazolyl-, Indolyl-, Chinolyl-, Benzofuranyl-, Benzo[b]thienyl-, Benzimidazolyl-, Benzoxazolyl- oder Benzothiazolylrest steht, wobei alle diese Reste gegebenenfalls durch einen oder mehrere gleiche oder verschiedene Substituenten substituiert sind, ausgewählt unter den Alkylresten mit 1 bis 5 Kohlenstoffatomen, den Alkoxyresten mit 1 bis 5 Kohlenstoffatomen, den Trifluormethyl-, Nitro-, Amino-, Monoalkyl- oder Dialkylaminoresten, deren Alkylgruppen 1 bis 5 Kohlenstoffatome aufweisen, und dem Phenylrest, der gegebenenfalls durch einen oder mehrere Alkylreste mit 1 bis 5 Kohlenstoffatomen, Alkoxyreste mit 1 bis 5 Kohlenstoffatomen oder Halogenen substituiert ist, wobei die Verbindungen der Formel (I) in sämtlichen möglichen enantiomeren und diastereomeren Formen und in Form der Additionssalze mit Säuren oder der quaternären Ammoniumsalze vorliegen können,
dadurch **gekennzeichnet,** daß man 8-Chlor-5,6,7,8-tetrahydrochinolin der Formel

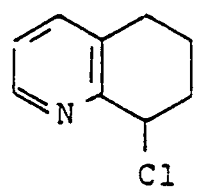

mit einem Amin der Formel

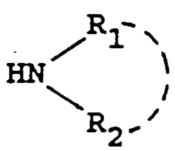

worin $R_1$ und $R_2$ die vorstehend angegebenen Bedeutungen besitzen, kondensiert, um zu einer Verbindung der Formel II

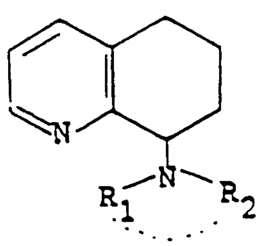

(II)

zu gelangen, welche man reduziert, um eine Verbindung der Formel III

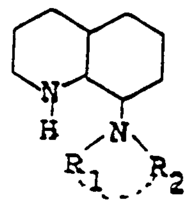

(III)

zu erhalten, die man mit einer Verbindung der Formel IV oder einem funktionellen Derivat dieser Verbindung

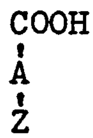

(IV)

worin A und Z die vorstehend angegebenen Bedeutungen besitzen, kondensiert, um zu einer Verbindung der Formel (I) in sämtlichen ihrer möglichen enantiomeren und diastereomeren Formen zu gelangen, die man gewünschtenfalls mit einer Mineral- oder organischen Säure behandelt, um ein Salz zu erhalten, oder mit einem Alkylhalogenid, um ein quaternäres Ammoniumsalz zu erhalten.

**2.** Verfahren zur Herstellung der Verbindungen der Formel (I), wie in Anspruch 1 definiert, dadurch gekennzeichnet, daß man die Reduktion der Verbindung der Formel II mit einem chemischen Reduktionsmittel durchführt und man bevorzugt die Verbindungen der Formel (I) erhält, worin die Anknüpfung des Rings trans-ständig ist, und daß man die Reduktion der Verbindung der Formel II durch katalytische Hydrierung bewirkt und vorzugsweise Verbindungen der Formel (I) erhält, worin die Anknüpfung des Rings cis-ständig ist.

**3.** Verfahren gemäß Anspruch 1, dadurch gekennzeichnet, daß man von einem Amin der Formel

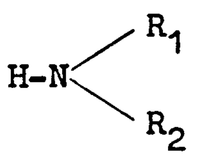

worin $R_1$ und $R_2$ für eine Methyl- oder Ethylgruppe stehen oder gemeinsam mit dem Stickstoffatom, an das sie gebunden sind, einen Pyrrolidinyl- oder Piperidinylrest bilden, und einer Verbindung der Formel (IV) oder einem funktionellen Derivat dieser Verbindung ausgeht, wobei in der Formel (IV) A eine Kette $(CH_2)_n$, worin n für 0 oder 1 steht, oder eine 1,1-Ethandiylkette wiedergibt, Z einen Phenylrest bedeutet, der gegebenenfalls durch einen oder mehrere gleiche oder verschiedene Reste substituiert ist, ausgewählt unter den Alkylresten mit 1 bis 5 Kohlenstoffatomen, den Alkoxyresten mit 1 bis 5 Kohlenstoffatomen, den Halogenatomen, den Hydroxyl-, Trifluormethyl-, Nitro-, Amino-, Monoalkyl- oder Dialkylaminoresten, deren Alkylgruppen 1 bis 5 Kohlenstoffatome aufweisen, oder Z einen Naphthylrest, einen Indenyl-, Pyridinyl-, Thienyl-, Thiazolyl-, Oxazolyl-, Isoxazolyl-, Imidazolyl-, Indolyl-, Chinolyl-, Benzofuranyl-, Benzo[b]thienyl-, Benzimidazolyl-, Benzoxazolyl- oder Benzothiazolylrest bedeutet, wobei alle diese Reste gegebenenfalls durch einen oder mehrere gleiche oder verschiedene Substituenten substituiert sind, ausgewählt unter den Alkylresten mit 1 bis 5 Kohlenstoffatomen, den Alkoxyresten mit 1 bis 5 Kohlenstoffatomen, den Trifluormethyl-, Nitro-, Amino-, Monoalkyl- oder Dialkylaminoresten, deren Alkylgruppen 1 bis 5 Kohlenstoffatome aufweisen, und dem Phenylrest, der gegebenenfalls durch einen oder mehrere Alkylreste mit 1 bis 5 Kohlenstoffatomen, Alkoxyreste mit 1 bis 5 Kohlenstoffatomen oder Halogene substituiert ist.

**4.** Verfahren gemäß Anspruch 3, dadurch gekennzeichnet, daß man von einer Verbindung der Formel (IV) oder einem funktionellen Derivat dieser Verbindung ausgeht, wobei in der Formel (IV) Z einen

Phenylrest bedeutet, der gegebenenfalls durch einen oder mehrere gleiche oder verschiedene Substituenten substituiert ist, ausgewählt unter den Alkylresten mit 1 bis 5 Kohlenstoffatomen, den Alkoxyresten mit 1 bis 5 Kohlenstoffatomen, den Halogenatomen, den Hydroxyl-, Trifluormethyl-, Nitro-, Amino-, Monoalkyl- oder Dialkylaminoresten, deren Alkylgruppen 1 bis 5 Kohlenstoffatome aufweisen, oder Z einen Naphthyl-, Pyridinyl-, Thienyl-, Indolyl-, Benzo[b]thienyl-Rest bedeutet, wobei alle diese Reste gegebenenfalls durch einen oder mehrere gleiche oder verschiedene Substituenten substituiert sind,ausgewählt unter den Alkylresten mit 1 bis 5 Kohlenstoffatomen, den Alkoxyresten mit 1 bis 5 Kohlenstoffatomen, den Trifluormethyl-, Nitro-, Amino-, Monoalkyl- oder Dialkylaminoresten, deren Alkylgruppen 1 bis 5 Kohlenstoffatome aufweisen, und dem Phenylrest, der gegebenenfalls durch einen oder mehrere Alkylreste mit 1 bis 5 Kohlenstoffatomen, Alkoxyreste mit 1 bis 5 Kohlenstoffatomen oder Halogene substituiert ist.

**5.** Verfahren gemäß einem der Ansprüche 1, 3 oder 4, dadurch gekennzeichnet, daß man von einem Amin der Formel

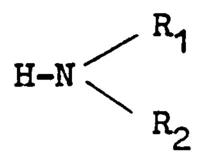

worin $R_1$ und $R_2$ gemeinsam mit dem Stickstoffatom, an das sie gebunden sind, einen Pyrrolidinylrest bilden, und einer Verbindung der Formel (IV) oder einem funktionellen Derivat dieser Verbindung ausgeht, wobei in der Formel (IV) A für eine Kette $(CH_2)_n$, worin n 0 oder 1 bedeutet, oder eine 1,1-Ethandiylkette steht, Z einen Phenylrest wiedergibt, der durch einen oder mehrere Substituenten, ausgewählt unter den Halogenatomen und der Trifluormethylgruppe, substituiert ist oder Z einen Naphthyl- oder Benzo[b]thienylrest bedeutet.

**6.** Verfahren gemäß Anspruch 5 zur Herstellung von (4aα,8α,8aα)-(±)-Decahydro-1-[(3,4-dichlorphenyl)-acetyl]-8-(1-pyrrolidinyl)-chinolin und dessen Additionssalzen mit Säuren, dadurch gekennzeichnet, daß man von einem Amin der Formel

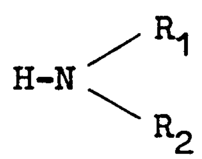

worin $R_1$ und $R_2$ gemeinsam mit dem Stickstoffatom, an das sie gebunden sind, einen Pyrrolidinylrest bilden, und einer Verbindung der Formel (IV) oder einem funktionellen Derivat dieser Verbindung ausgeht, wobei in der Formel (IV) A für eine Kette $-CH_2-$ steht und Z einen 3,4-Dichlorphenylrest bedeutet.

**7.** Verfahren gemäß Anspruch 5 zur Herstellung von Verbindungen der Formel (I), wie in Anspruch 1 definiert, mit den folgenden Bezeichnungen:

(4aα, 8α,8aα)-(±)-Decahydro-1-[[4-(trifluormethyl)-phenyl]-acetyl]-8-(1-pyrrolidinyl)-chinolin,
(4aα,8α,8aα)-(±)-Decahydro-1-[(4-bromphenyl)-acetyl]-8-(1-pyrrolidinyl)-chinolin,
(4aα,8α,8aα)-(±)-Decahydro-1-[2-(3,4-dichlorphenyl)-1-oxopropyl]-8-(1-pyrrolidinyl)-chinolin,
(4aα,8α,8aα)-(±)-Decahydro-1-(3,4-dichlorbenzoyl)-8-(1-pyrrolidinyl)-chinolin,
(4aα,8α,8aα)-(±)-Decahydro-1-[(4-benzo[b]thienyl)-acetyl]-8-(1-pyrrolidinyl)-chinolin,
(4aα,8α,8aα)-(±)-Decahydro-1-[(1-naphthalinyl)-acetyl]-8-(1-pyrrolidinyl)-chinolin,

sowie von deren Additionssalzen mit Säuren und deren quaternären Ammoniumsalzen, dadurch gekennzeichnet, daß man von einem Amin der Formel

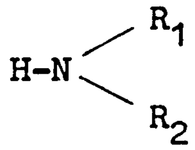

worin $R_1$ und $R_2$ gemeinsam mit dem Stickstoffatom, an das sie gebunden sind, einen Pyrrolidinylrest bilden, und einer Verbindung der Formel (IV) oder einem funktionellen Derivat dieser Verbindung ausgeht, wobei in der Formel (IV) A und Z die den vorstehend bezeichneten Verbindungen entsprechenden Bedeutungen besitzen.

**Patentansprüche für folgenden Vertragsstaat : ES**

1. Verfahren zur Herstellung der Verbindungen der Formel (I)

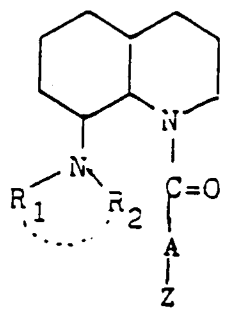

(I)

worin $R_1$ und $R_2$, die gleich oder verschieden sind, ein Wasserstoffatom oder einen Alkylrest mit 1 bis 5 Kohlenstoffatomen bedeuten oder $R_1$ und $R_2$ gemeinsam mit dem Stickstoffatom, an das sie gebunden sind, einen Pyrrolidinyl-, Piperazinyl-, Piperidinyl- oder Morpholinylrest bilden, wobei diese Reste gegebenenfalls durch einen Alkyl-oder Alkoxyrest mit 1 bis 5 Kohlenstoffatomen substituiert sind; A eine Kette $(CH_2)_n$ wiedergibt, worin n eine Zahl von 0 bis 5 bedeutet, oder A eine durch einen Alkylrest mit insgesamt 2 bis 8 Kohlenstoffatomen substituierte Alkylenkette bedeutet; Z für einen Phenylrest steht, der gegebenenfalls durch einen oder mehrere gleiche oder verschiedene Reste substituiert ist, ausgewählt unter den Alkylresten mit 1 bis 5 Kohlenstoffatomen, den Alkoxyresten mit 1 bis 5 Kohlenstoffatomen, den Halogenatomen, den Hydroxyl-, Trifluormethyl-, Nitro-, Amino-, Monoalkyl- oder Dialkylaminoresten, deren Alkylgruppen 1 bis 5 Kohlenstoffatome enthalten, oder Z für einen Naphthyl-, einen Indenyl-, Pyridinyl-, Thienyl-, Thiazolyl-, Oxazolyl-, Isoxazolyl-, Imidazolyl-, Indolyl-, Chinolyl-, Benzofuranyl-, Benzo[b]thienyl-, Benzimidazolyl-, Benzoxazolyl- oder Benzothiazolylrest steht, wobei alle diese Reste gegebenenfalls durch einen oder mehrere gleiche oder verschiedene Substituenten substituiert sind, ausgewählt unter den Alkylresten mit 1 bis 5 Kohlenstoffatomen, den Alkoxyresten mit 1 bis 5 Kohlenstoffatomen, den Trifluormethyl-, Nitro-, Amino-, Monoalkyl- oder Dialkylaminoresten, deren Alkylgruppen 1 bis 5 Kohlenstoffatome aufweisen, und dem Phenylrest, der gegebenenfalls durch einen oder mehrere Alkylreste mit 1 bis 5 Kohlenstoffatomen, Alkoxyreste mit 1 bis 5 Kohlenstoffatomen oder Halogenen substituiert ist, wobei die Verbindungen der Formel (I) in sämtlichen möglichen enantiomeren und diastereomeren Formen und in Form der Additionssalze mit Säuren oder der quaternären Ammoniumsalze vorliegen können,
dadurch **gekennzeichnet,** daß man 8-Chlor-5,6,7,8-tetrahydrochinolin der Formel

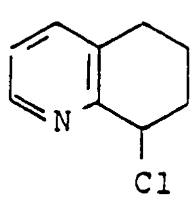

mit einem Amin der Formel

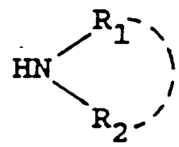

worin $R_1$ und $R_2$ die vorstehend angegebenen Bedeutungen besitzen, kondensiert, um zu einer Verbindung der Formel II

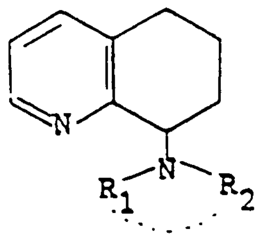

(II)

zu gelangen, welche man reduziert, um eine Verbindung der Formel III

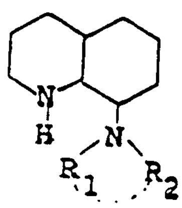

(III)

zu erhalten, die man mit einer Verbindung der Formel IV oder einem funktionellen Derivat dieser Verbindung

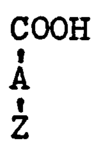

(IV)

worin A und Z die vorstehend angegebenen Bedeutungen besitzen, kondensiert, um zu einer Verbindung der Formel (I) in sämtlichen ihrer möglichen enantiomeren und diastereomeren Formen zu gelangen, die man gewünschtenfalls mit einer Mineral- oder organischen Säure behandelt, um ein Salz zu erhalten, oder mit einem Alkylhalogenid, um ein quaternäres Ammoniumsalz zu erhalten.

**2.** Verfahren zur Herstellung der Verbindungen der Formel (I), wie in Anspruch 1 definiert, dadurch gekennzeichnet, daß man die Reduktion der Verbindung der Formel II mit einem chemischen Reduktionsmittel durchführt und man bevorzugt die Verbindungen der Formel (I) erhält, worin die Anknüpfung des Rings trans-ständig ist, und daß man die Reduktion der Verbindung der Formel II durch katalytische Hydrierung bewirkt und vorzugsweise Verbindungen der Formel (I) erhält, worin die Anknüpfung des Rings cis-ständig ist.

**3.** Verfahren gemäß Anspruch 1, dadurch gekennzeichnet, daß man von einem Amin der Formel

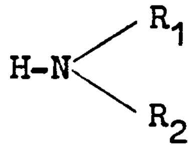

worin $R_1$ und $R_2$ für eine Methyl- oder Ethylgruppe stehen oder gemeinsam mit dem Stickstoffatom, an das sie gebunden sind, einen Pyrrolidinyl- oder Piperidinylrest bilden, und einer Verbindung der Formel (IV) oder einem funktionellen Derivat dieser Verbindung ausgeht, wobei in der Formel (IV) A eine Kette $(CH_2)_n$, worin n für 0 oder 1 steht, oder eine 1,1-Ethandiylkette wiedergibt, Z einen Phenylrest bedeutet, der gegebenenfalls durch einen oder mehrere gleiche oder verschiedene Reste substituiert ist, ausgewählt unter den Alkylresten mit 1 bis 5 Kohlenstoffatomen, den Alkoxyresten mit 1 bis 5 Kohlenstoffatomen, den Halogenatomen, den Hydroxyl-, Trifluormethyl-, Nitro-, Amino-, Monoalkyl oder Dialkylaminoresten, deren Alkylgruppen 1 bis 5 Kohlenstoffatome aufweisen, oder Z einen Naphthylrest, einen Indenyl-, Pyridinyl-, Thienyl-, Thiazolyl-, Oxazolyl-, Isoxazolyl-, Imidazolyl-, Indolyl-, Chinolyl-, Benzofuranyl-, Benzo[b]thienyl-, Benzimidazolyl-, Benzoxazolyl- oder Benzothiazolylrest bedeutet, wobei alle diese Reste gegebenenfalls durch einen oder mehrere gleiche oder verschiedene Substituenten substituiert sind, ausgewählt unter den Alkylresten mit 1 bis 5 Kohlenstoffatomen, den Alkoxyresten mit 1 bis 5 Kohlenstoffatomen, den Trifluormethyl-, Nitro-, Amino-, Monoalkyl- oder Dialkylaminoresten, deren Alkylgruppen 1 bis 5 Kohlenstoffatome aufweisen, und dem Phenylrest, der gegebenenfalls durch einen oder mehrere Alkylreste mit 1 bis 5 Kohlenstoffatomen, Alkoxyreste mit 1 bis 5 Kohlenstoffatomen oder Halogene substituiert ist.

**4.** Verfahren gemäß Anspruch 3, dadurch gekennzeichnet, daß man von einer Verbindung der Formel (IV) oder einem funktionellen Derivat dieser Verbindung ausgeht, wobei in der Formel (IV) Z einen Phenylrest bedeutet, der gegebenenfalls durch einen oder mehrere gleiche oder verschiedene Substituenten substituiert ist, ausgewählt unter den Alkylresten mit 1 bis 5 Kohlenstoffatomen, den Alkoxyresten mit 1 bis 5 Kohlenstoffatomen, den Halogenatomen, den Hydroxyl-, Trifluormethyl-, Nitro-, Amino-, Monoalkyl- oder Dialkylaminoresten, deren Alkylgruppen 1 bis 5 Kohlenstoffatome aufweisen, oder Z einen Naphthyl-, Pyridinyl-, Thienyl-, Indolyl-, Benzo[b]thienyl-Rest bedeutet, wobei alle diese Reste gegebenenfalls durch einen oder mehrere gleiche oder verschiedene Substituenten substituiert sind,ausgewählt unter den Alkylresten mit 1 bis 5 Kohlenstoffatomen, den Alkoxyresten mit 1 bis 5 Kohlenstoffatomen, den Trifluormethyl-, Nitro-, Amino-, Monoalkyl- oder Dialkylaminoresten, deren Alkylgruppen 1 bis 5 Kohlenstoffatome aufweisen, und dem Phenylrest, der gegebenenfalls durch einen oder mehrere Alkylreste mit 1 bis 5 Kohlenstoffatomen, Alkoxyreste mit 1 bis 5 Kohlenstoffatomen oder Halogene substituiert ist.

**5.** Verfahren gemäß einem der Ansprüche 1, 3 oder 4, dadurch gekennzeichnet, daß man von einem Amin der Formel

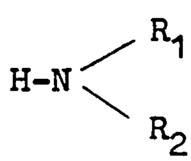

worin $R_1$ und $R_2$ gemeinsam mit dem Stickstoffatom, an das sie gebunden sind, einen Pyrrolidinylrest bilden, und einer Verbindung der Formel (IV) oder einem funktionellen Derivat dieser Verbindung ausgeht, wobei in der Formel (IV) A für eine Kette $(CH_2)_n$, worin n 0 oder 1 bedeutet, oder eine 1,1-Ethandiylkette steht, Z einen Phenylrest wiedergibt, der durch einen oder mehrere Substituenten, ausgewählt unter den Halogenatomen und der Trifluormethylgruppe, substituiert ist oder Z einen Naphthyl- oder Benzo[b]thienylrest bedeutet.

**6.** Verfähren gemäß Anspruch 5 zur Herstellung von (4aα,8α,8aα)-(±)-Decahydro-1-[(3,4-dichlorphenyl)-acetyl]-8-(1-pyrrolidinyl)-chinolin und dessen Additionssalzen mit Säuren, dadurch gekennzeichnet, daß man von einem Amin der Formel

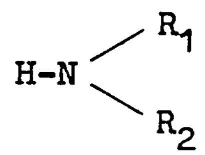

worin $R_1$ und $R_2$ gemeinsam mit dem Stickstoffatom, an das sie gebunden sind, einen Pyrrolidinylrest bilden, und einer Verbindung der Formel (IV) oder einem funktionellen Derivat dieser Verbindung ausgeht, wobei in der Formel (IV) A für eine Kette $-CH_2-$ steht und Z einen 3,4-Dichlorphenylrest bedeutet.

**7.** Verfahren gemäß Anspruch 5 zur Herstellung von Verbindungen der Formel (I), wie in Anspruch 1 definiert, mit den folgenden Bezeichnungen:
(4aα, 8α,8aα)-(±)-Decahydro-1-[[4-(trifluormethyl)-phenyl]-acetyl]-8-(1-pyrrolidinyl)-chinolin,
(4aα,8α,8aα)-(±)-Decahydro-1-[(4-bromphenyl)-acetyl]-8-(1-pyrrolidinyl)-chinolin,
(4aα,8α,8aα)-(±)-Decahydro-1-[2-(3,4-dichlorphenyl)-1-oxopropyl]-8-(1-pyrrolidinyl)-chinolin,
(4aα,8α,8aα)-(±)-Decahydro-1-(3,4-dichlorbenzoyl)-8-(1-pyrrolidinyl)-chinolin,
(4aα,8α,8aα)-(±)-Decahydro-1-[(4-benzo[b]thienyl)-acetyl]-8-(1-pyrrolidinyl)-chinolin,
(4aα,8α,8aα)-(±)-Decahydro-1-[(1-naphthalinyl)-acetyl]-8-(1-pyrrolidinyl)-chinolin,
sowie von deren Additionssalzen mit Säuren und deren quaternären Ammoniumsalzen, dadurch gekennzeichnet, daß man von einem Amin der Formel

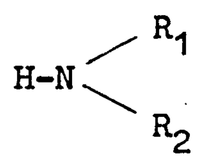

worin $R_1$ und $R_2$ gemeinsam mit dem Stickstoffatom, an das sie gebunden sind, einen Pyrrolidinylrest bilden, und einer Verbindung der Formel (IV) oder einem funktionellen Derivat dieser Verbindung ausgeht, wobei in der Formel (IV) A und Z die den vorstehend bezeichneten Verbindungen entsprechenden Bedeutungen besitzen.

**Patentansprüche für folgenden Vertragsstaat : GR**

**1.** Verfahren zur Herstellung der Verbindungen der Formel (I)

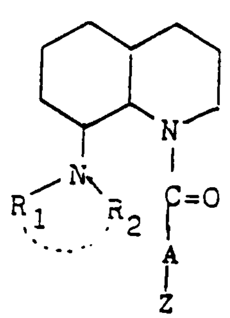

(I)

worin $R_1$ und $R_2$, die gleich oder verschieden sind, ein Wasserstoffatom oder einen Alkylrest mit 1 bis 5 Kohlenstoffatomen bedeuten oder $R_1$ und $R_2$ gemeinsam mit dem Stickstoffatom, an das sie gebunden sind, einen Pyrrolidinyl-, Piperazinyl-, Piperidinyl- oder Morpholinylrest bilden, wobei diese Reste gegebenenfalls durch einen Alkyl-oder Alkoxyrest mit 1 bis 5 Kohlenstoffatomen substituiert sind; A eine Kette $(CH_2)_n$ wiedergibt, worin n eine Zahl von 0 bis 5 bedeutet, oder A eine durch einen Alkylrest mit insgesamt 2 bis 8 Kohlenstoffatomen substituierte Alkylenkette bedeutet; Z für einen Phenylrest steht, der gegebenenfalls durch einen oder mehrere gleiche oder verschiedene Reste

substituiert ist, ausgewählt unter den Alkylresten mit 1 bis 5 Kohlenstoffatomen, den Alkoxyresten mit 1 bis 5 Kohlenstoffatomen, den Halogenatomen, den Hydroxyl-, Trifluormethyl-, Nitro-, Amino-, Monoalkyl- oder Dialkylaminoresten, deren Alkylgruppen 1 bis 5 Kohlenstoffatome enthalten, oder Z für einen Naphthyl-, einen Indenyl-, Pyridinyl-, Thienyl-, Thiazolyl-, Oxazolyl-, Isoxazolyl-, Imidazolyl-, Indolyl-, Chinolyl-, Benzofuranyl-, Benzo[b]thienyl, Benzimidazolyl-, Benzoxazolyl- oder Benzothiazolylrest steht, wobei alle diese Reste gegebenenfalls durch einen oder mehrere gleiche oder verschiedene Substituenten substituiert sind, ausgewählt unter den Alkylresten mit 1 bis 5 Kohlenstoffatomen, den Alkoxyresten mit 1 bis 5 Kohlenstoffatomen, den Trifluormethyl-, Nitro-, Amino-, Monoalkyl- oder Dialkylamino-resten, deren Alkylgruppen 1 bis 5 Kohlenstoffatome aufweisen, und dem Phenylrest, der gegebenenfalls durch einen oder mehrere Alkylreste mit 1 bis 5 Kohlenstoffatomen, Alkoxyreste mit 1 bis 5 Kohlenstoffatomen oder Halogenen substituiert ist, wobei die Verbindungen der Formel (I) in sämtlichen möglichen enantiomeren und diastereomeren Formen und in Form der Additionssalze mit Säuren oder der quaternären Ammoniumsalze vorliegen können,
dadurch **gekennzeichnet,** daß man 8-Chlor-5,6,7,8-tetrahydrochinolin der Formel

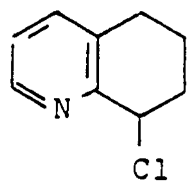

mit einem Amin der Formel

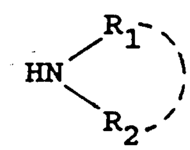

worin $R_1$ und $R_2$ die vorstehend angegebenen Bedeutungen besitzen, kondensiert, um zu einer Verbindung der Formel II

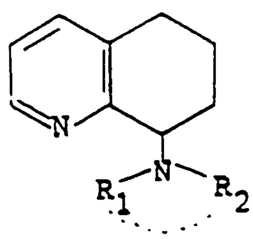

(II)

zu gelangen, welche man reduziert, um eine Verbindung der Formel III

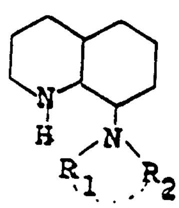

(III)

zu erhalten, die man mit einer Verbindung der Formel IV oder einem funktionellen Derivat dieser Verbindung

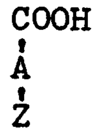

(IV)

worin A und Z die vorstehend angegebenen Bedeutungen besitzen, kondensiert, um zu einer Verbindung der Formel (I) in sämtlichen ihrer möglichen enantiomeren und diastereomeren Formen zu gelangen, die man gewünschtenfalls mit einer Mineral- oder organischen Säure behandelt, um ein Salz zu erhalten, oder mit einem Alkylhalogenid, um ein quaternäres Ammoniumsalz zu erhalten.

**2.** Verfahren zur Herstellung der Verbindungen der Formel (I), wie in Anspruch 1 definiert, dadurch gekennzeichnet, daß man die Reduktion der Verbindung der Formel II mit einem chemischen Reduktionsmittel durchführt und man bevorzugt die Verbindungen der Formel (I) erhält, worin die Anknüpfung des Rings trans-ständig ist, und daß man die Reduktion der Verbindung der Formel II durch katalytische Hydrierung bewirkt und vorzugsweise Verbindungen der Formel (I) erhält, worin die Anknüpfung des Rings cis-ständig ist.

**3.** Verfahren gemäß Anspruch 1, dadurch gekennzeichnet, daß man von einem Amin der Formel

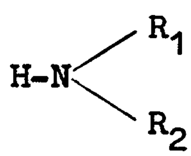

worin $R_1$ und $R_2$ für eine Methyl- oder Ethylgruppe stehen oder gemeinsam mit dem Stickstoffatom, an das sie gebunden sind, einen Pyrrolidinyl- oder Piperidinylrest bilden, und einer Verbindung der Formel (IV) oder einem funktionellen Derivat dieser Verbindung ausgeht, wobei in der Formel (IV) A eine Kette $(CH_2)_n$, worin n für 0 oder 1 steht, oder eine 1,1-Ethandiylkette wiedergibt, Z einen Phenylrest bedeutet, der gegebenenfalls durch einen oder mehrere gleiche oder verschiedene Reste substituiert ist, ausgewählt unter den Alkylresten mit 1 bis 5 Kohlenstoffatomen, den Alkoxyresten mit 1 bis 5 Kohlenstoffatomen, den Halogenatomen, den Hydroxyl-, Trifluormethyl-, Nitro-, Amino-, Monoalkyl- oder Dialkylaminoresten, deren Alkylgruppen 1 bis 5 Kohlenstoffatome aufweisen, oder Z einen Naphthylrest, einen Indenyl-, Pyridinyl-, Thienyl-, Thiazolyl-, Oxazolyl-, Isoxazolyl-, Imidazolyl-, Indolyl-, Chinolyl-, Benzofuranyl-, Benzo[b]thienyl-, Benzimidazolyl-, Benzoxazolyl- oder Benzothiazolylrest bedeutet, wobei alle diese Reste gegebenenfalls durch einen oder mehrere gleiche oder verschiedene Substituenten substituiert sind, ausgewählt unter den Alkylresten mit 1 bis 5 Kohlenstoffatomen, den Alkoxyresten mit 1 bis 5 Kohlenstoffatomen, den Trifluormethyl-, Nitro-, Amino-, Monoalkyl- oder Dialkylaminoresten, deren Alkylgruppen 1 bis 5 Kohlenstoffatome aufweisen, und dem Phenylrest, der gegebenenfalls durch einen oder mehrere Alkylreste mit 1 bis 5 Kohlenstoffatomen, Alkoxyreste mit 1 bis 5 Kohlenstoffatomen oder Halogene substituiert ist.

**4.** Verfahren gemäß Anspruch 3, dadurch gekennzeichnet, daß man von einer Verbindung der Formel (IV) oder einem funktionellen Derivat dieser Verbindung ausgeht, wobei in der Formel (IV) Z einen Phenylrest bedeutet, der gegebenenfalls durch einen oder mehrere gleiche oder verschiedene Substituenten substituiert ist, ausgewählt unter den Alkylresten mit 1 bis 5 Kohlenstoffatomen, den Alkoxyresten mit 1 bis 5 Kohlenstoffatomen, den Halogenatomen, den Hydroxyl-, Trifluormethyl-, Nitro-, Amino-, Monoalkyl- oder Dialkylaminoresten, deren Alkylgruppen 1 bis 5 Kohlenstoffatome aufweisen, oder Z einen Naphthyl-, Pyridinyl-, Thienyl-, Indolyl-, Benzo[b]thienyl-Rest bedeutet, wobei alle diese Reste gegebenenfalls durch einen oder mehrere gleiche oder verschiedene Substituenten substituiert sind,ausgewählt unter den Alkylresten mit 1 bis 5 Kohlenstoffatomen, den Alkoxyresten mit 1 bis 5 Kohlenstoffatomen, den Trifluormethyl-, Nitro-, Amino-, Monoalkyl- oder Dialkylaminoresten, deren Alkylgruppen 1 bis 5 Kohlenstoffatome aufweisen, und dem Phenylrest, der gegebenenfalls durch einen oder mehrere Alkylreste mit 1 bis 5 Kohlenstoffatomen, Alkoxyreste mit 1 bis 5 Kohlenstoffatomen oder Halogene substituiert ist.

5. Verfahren gemäß einem der Ansprüche 1, 3 oder 4, dadurch gekennzeichnet, daß man von einem Amin der Formel

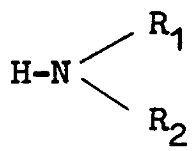

worin $R_1$ und $R_2$ gemeinsam mit dem Stickstoffatom, an das sie gebunden sind, einen Pyrrolidinylrest bilden, und einer Verbindung der Formel (IV) oder einem funktionellen Derivat dieser Verbindung ausgeht, wobei in der Formel (IV) A für eine Kette $(CH_2)_n$, worin n 0 oder 1 bedeutet, oder eine 1,1-Ethandiylkette steht, Z einen Phenylrest wiedergibt, der durch einen oder mehrere Substituenten, ausgewählt unter den Halogenatomen und der Trifluormethylgruppe, substituiert ist oder Z einen Naphthyl- oder Benzo[b]thienylrest bedeutet.

6. Verfahren gemäß Anspruch 5 zur Herstellung von (4aα,8α,8aα)-(±)-Decahydro-1-[(3,4-dichlorphenyl)-acetyl]-8-(1-pyrrolidinyl)-chinolin und dessen Additionssalzen mit Säuren, dadurch gekennzeichnet, daß man von einem Amin der Formel

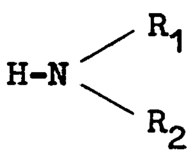

worin $R_1$ und $R_2$ gemeinsam mit dem Stickstoffatom, an das sie gebunden sind, einen Pyrrolidinylrest bilden, und einer Verbindung der Formel (IV) oder einem funktionellen Derivat dieser Verbindung ausgeht, wobei in der Formel (IV) A für eine Kette $-CH_2-$ steht und Z einen 3,4-Dichlorphenylrest bedeutet.

7. Verfahren gemäß Anspruch 5 zur Herstellung von Verbindungen der Formel (I), wie in Anspruch 1 definiert, mit den folgenden Bezeichnungen:
   (4aα, 8α,8aα)-(±)-Decahydro-1-[[4-(trifluormethyl)-phenyl]-acetyl]-8-(1-pyrrolidinyl)-chinolin,
   (4aα,8α,8aα)-(±)-Decahydro-1-[(4-bromphenyl)-acetyl]-8-(1-pyrrolidinyl)-chinolin,
   (4aα,8α,8aα)-(±)-Decahydro-1-[2-(3,4-dichlorphenyl)-1-oxopropyl]-8-(1-pyrrolidinyl)-chinolin,
   (4aα,8α,8aα)-(±)-Decahydro-1-(3,4-dichlorbenzoyl)-8-(1-pyrrolidinyl)-chinolin,
   (4aα,8α,8aα)-(±)-Decahydro-1-[(4-benzo[b]thienyl)-acetyl]-8-(1-pyrrolidinyl)-chinolin,
   (4aα,8α,8aα)-(±)-Decahydro-1-[(1-naphthalinyl)-acetyl]-8-(1-pyrrolidinyl)-chinolin,
   sowie von deren Additionssalzen mit Säuren und deren quaternären Ammoniumsalzen, dadurch gekennzeichnet, daß man von einem Amin der Formel

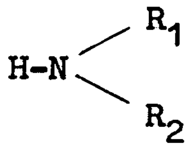

worin $R_1$ und $R_2$ gemeinsam mit dem Stickstoffatom, an das sie gebunden sind, einen Pyrrolidinylrest bilden, und einer Verbindung der Formel (IV) oder einem funktionellen Derivat dieser Verbindung ausgeht, wobei in der Formel (IV) A und Z die den vorstehend bezeichneten Verbindungen entsprechenden Bedeutungen besitzen.

8. Als neues, industrielles Produkt das 8-Chlor-5,6,7,8-tetrahydrochinolin.

9. Als neue, industrielle Produkte die Produkte der Formeln II und III, wie in Anspruch 1 definiert.

**10.** Verfahren zur Herstellung von pharmazeutischen Zusammensetzungen, dadurch gekennzeichnet, daß man als Wirkstoff zumindest eine der Verbindungen der Formel (I), wie in Anspruch 1 definiert, oder zumindest eines ihrer pharmazeutisch verträglichen Salze in eine für diese Verwendung vorgesehene Form bringt.

**11.** Verfahren zur Herstellung von pharmazeutischen Zusammensetzungen, dadurch gekennzeichnet, daß man als Wirkstoff zumindest eine der Verbindungen der Formel (I), wie in Anspruch 2 definiert, oder zumindest eines ihrer pharmazeutisch verträglichen Salze in eine für diese Verwendung vorgesehene Form bringt.

**12.** Verfahren zur Herstellung von pharmazeutischen Zusammensetzungen, dadurch gekennzeichnet, daß man als Wirkstoff zumindest eine der Verbindungen der Formel (I), wie in den Ansprüchen 3, 4 oder 5 definiert, oder zumindest eines ihrer pharmazeutisch verträglichen Salze in eine für diese Verwendung vorgesehene Form bringt.

**13.** Verfahren gemäß Anspruch 10, dadurch gekennzeichnet, daß der Wirkstoff ausgewählt wird unter
(4aα,8α,8aα)-(±)-Decahydro-1-[(4-(trifluormethyl)-phenyl]-acetyl]-8-(1-pyrrolidinyl)-chinolin,
(4aα,8α,8aα)-(±)-Decahydro-1-[(4-bromphenyl)-acetyl]-8-(1-pyrrolidinyl)-chinolin,
(4aα,8α,8aα)-(±)-Decahydro-1-[2-(3,4-dichlorphenyl)-1-oxopropyl]-8-(1-pyrrolidinyl)-chinolin,
(4aα,8α,8aα)-(±)-Decahydro-1-(3,4-dichlorbenzoyl)-8-(1-pyrrolidinyl)-chinolin,
(4aα,8α,8aα)-(±)-Decahydro-1-[(4-benzo[b]thienyl)-acetyl]-8-(1-pyrrolidinyl)-chinolin,
(4aα,8α,8aα)-(±)-Decahydro-1-[(1-naphthalinyl)-acetyl]-8-(1-pyrrolidinyl)-chinolin,
sowie deren Additionssalzen mit Säuren und deren quaternären Ammoniumsalzen.

**14.** Verfahren gemäß Anspruch 10, dadurch gekennzeichnet, daß der Wirkstoff aus (4aα,8α,8aα)-(±)-Decahydro-1-[(3,4-dichlorphenyl)-acetyl]-8-(1-pyrrolidinyl)-chinolin oder einem seiner Additionssalze mit Säuren besteht.